(19) Europäisches Patentamt
European Patent Office
Office européen des brevets

(11) Publication number: **0 029 363**
**A1**

(12) # EUROPEAN PATENT APPLICATION

(21) Application number: **80304106.0**

(22) Date of filing: **14.11.80**

(51) Int. Cl.³: **C 07 D 231/14**, C 07 D 231/12, A 61 K 31/415

(30) Priority: **16.11.79 JP 149099/79**
**16.11.79 JP 149100/79**
**16.11.79 JP 149101/79**
**16.11.79 JP 149102/79**

(43) Date of publication of application: **27.05.81**
**Bulletin 81/21**

(84) Designated Contracting States: **AT BE CH DE FR GB IT LI NL SE**

(71) Applicant: **MORISHITA PHARMACEUTICAL CO. LTD.,**
**29 Doshomachi 4-chome Higashi-ku, Osaka (JP)**

(72) Inventor: **Seki, Kunio, 1823-1 Ohshinohara, Yasu-cho Yasu-gun, Shiga-ken (JP)**
Inventor: **Ohki, Masahiko, 720-77 Toba-Otsu, Yasu-cho Yasu-gun, Shiga-ken (JP)**

(74) Representative: **Myerscough, Philip Boyd et al, J.A.Kemp & Co. 14, South Square Gray's Inn, London, WC1R 5EU (GB)**

㊹ **Pyrazole derivatives and pharmaceutical compositions containing them.**

㊼ Pyrazole derivatives of the general formula:

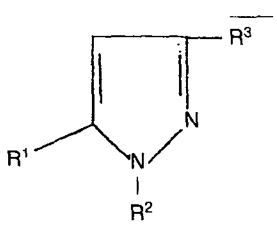

wherein $R^1$ represents an alkyl group of 7 to 17 carbon atoms, $R^2$ represents a hydrogen atom, a lower alkyl group or a phenyl group and $R^3$ represents $-\underset{|}{C}=O$ (with $OR^4$), $-\underset{|}{C}=O$ (with $NH-R^5$) or $-CH_2OH$, wherein $R^4$ represents a hydrogen atom or a lower alkyl group, $R^5$ represents $-CH_2CH_2OH$ or $-CH_2-\underset{|}{C}=O$ (with $R^6$), wherein $R^6$ represents a hydroxyl group or a lower alkoxy group with a proviso that when $R^1$ represents an alkyl group 7 to 11 carbon atoms and $R^2$ represents a hydrogen atom, $R^3$ does not represent $-\underset{|}{C}=O$ (with $OR^4$), are novel compounds which have excellent antilipemic activity with a very low toxicity to humans, and accordingly are suitable for use as pharmaceutical agents in the treatment of lipemia.

EP 0 029 363 A1

DESCRIPTION

"PYRAZOLE DERIVATIVES AND PHARMACEUTICAL COMPOSITIONS CONTAINING THEM"

The present invention relates to novel pyrazole derivatives having excellent antilipemic activity and suitable for use in the treatment of lipemia.

Although several antilipemic pharmaceutical agents have been proposed for use in the treatment of lipemia, they suffer the common defect of exhibiting side effects upon long term administration. For instance, clofibrate which has been widely administered as an antilipemic agent causes diseases in the biliary duct and there is the possibility that clofibrate may cause cancers. Further, pharmaceutical compositions comprising nicotinic acid are only rarely used because of the large dosages required and the side effect of causing flushing and eruptions on the face and skin of the patient being treated.

After synthesizing and testing various compounds we have found that pyrazole derivatives of the general formula (I) shown below have excellent antilipemic activity.

Pyrazole derivatives of the general formula (II):

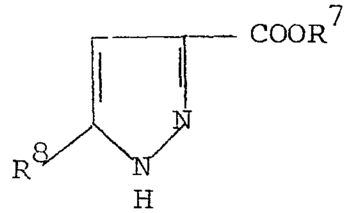

(II)

wherein $R^7$ represents a hydrogen atom or a lower alkyl

group and $R^8$ represents an alkyl group of 3 to 11 carbon atoms, are known, and their synthesis and their physical- and chemical properties have been reported by Keshin, H et al. (Istanbul Univ. Fen. Fak. Mecum. Seric., 34, 95-108, 1969), but without mention of any pharmacological properties.

Although Kupiecki F. et al reported that 5-methylpyrazole-3-carboxylic acid can reduce the level of lipid in human blood (refer to J. Pharmacol. Expt. Therap., 160, 166, 1968), Lang P.D. et al subsequently reported that its clinical effectiveness was not decisive and its use caused side effects such as hypoglycemia, rendering it unsuitable for use in treating lipemia (refer to Arzneim. Forsch., 25(1), 117, 1975).

Accordingly, the fact that the pyrazole derivatives of the present invention have excellent antilipemic activity and that they could safely be used in the treatment of lipemia was not to be expected from the prior art.

The pyrazole derivatives of the present invention are represented by the general formula (I)

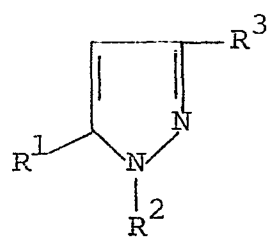

(I)

wherein $R^1$ represents an alkyl group of 7 to 17 carbon atoms, $R^2$ represents a hydrogen atom, a lower (preferably $C_1$-$C_4$) alkyl group or a phenyl group, $R^3$ represents $-\underset{OR^4}{\underset{|}{C}}{=}O$, $-\underset{NH-R^5}{\underset{|}{C}}{=}O$ or $-CH_2OH$ wherein $R^4$ represents a hydrogen atom or a lower (preferably

BAD ORIGINAL

$C_1$-$C_4$) alkyl group, $R^5$ represents $-CH_2CH_2OH$ or $-CH_2-\underset{R^6}{\underset{|}{C}}=O$ wherein $R^6$ represents a hydroxyl group or a lower (preferably $C_1$-$C_4$) alkoxy group, with the proviso that when $R^1$ represents an alkyl group of 7 to 11 carbon atoms and $R^2$ represents a hydrogen atom, $R^3$ does not represent $-\underset{OR^4}{\underset{|}{C}}=O$.

Examples of pyrazole derivatives of formula (I) (hereinafter referred to as the present pyrazole derivatives) are: 5-n-tridecylpyrazole-3-carboxylic acid, 5-n-pentadecylpyrazole-3-carboxylic acid, 5-n-heptadecylpyrazole-3-carboxylic acid, ethyl 5-n-tridecylpyrazole-3-carboxylate, ethyl 5-n-pentadecylpyrazole-3-carboxylate, ethyl 5-n-heptadecylpyrazole-3-carboxylate, 3-N-2'-hydroxyethylcarbamoyl-5-n-nonylpyrazole, 3-N-2'-hydroxyethylcarbamoyl-5-n-undecylpyrazole, 3-N-2'-hydroxyethylcarbamoyl-5-n-tridecylpyrazole, 3-N-hydroxycarbonylmethylcarbamoyl-5-n-nonylpyrazole, 3-N-hydroxycarbonylmethylcarbamoyl-5-n-undecylpyrazole, 3-N-hydroxycarbonylmethyl-5-n-tridecylpyrazole, 3-N-ethoxycarbonylmethylcarbamoyl-5-n-nonylpyrazole, 3-N-ethoxycarbonylmethylcarbamoyl-5-n-undecylpyrazole, 3-N-ethoxycarbonylmethylcarbamoyl-5-n-tridecylpyrazole, 3-hydroxymethyl-5-n-tridecylpyrazole, 3-hydroxymethyl-5-n-heptylpyrazole, 3-hydroxymethyl-5-n-nonylpyrazole, 3-hydroxymethyl-5-n-undecylpyrazole, 1-methyl-5-n-tridecylpyrazole-3-carboxylic acid, 1-methyl-5-n-heptapyrazole-3-carboxylic acid, 1-methyl-5-n-nonylpyrazole-3-carboxylic acid, 1-methyl-n-undecylpyrazole-3-carboxylic acid, 1-phenyl-5-n-nonylpyrazole-3-carboxylic acid, 1-phenyl-5-n-heptylpyrazole-3-carboxylic acid, 1-phenyl-5-n-undecyl-

pyrazole-3-carboxylic acid and 1-phenyl-5-n-tridecyl-pyrazole-3-carboxylic acid.

The present pyrazole derivatives can be synthesized by the following methods:

(1) Syntheses of 5-alkylpyrazole-3-carboxylic acids and esters thereof:

A sodium enolate of the general formula (III) obtainable by a Claisen condensation reaction between a higher-alkyl methyl ketone and diethyl oxalate:

$$R^{1}-\underset{O}{\underset{\|}{C}}-CH=\underset{ONa}{\underset{|}{C}}-CO_2C_2H_5 \qquad (III)$$

wherein $R^1$ is as defined for formula (I), is reacted with hydrazine hydrate in a mineral acid for 4 to 10 hours with heating and stirring under reflux to provide an ethyl ester of a 5-alkylpyrazole-3-carboxylic acid.

The corresponding acid can be obtained by hydrolyzing the ethyl ester with sodium hydroxide in an aqueous lower alcoholic solution at a temperature around 100°C, or by reacting a sodium enolate (III) with hydrazine sulfate in an aqueous lower alcoholic solution of sodium hydroxide with heating under reflux for 2 to 3 hours.

(2) Synthesis of 5-alkylpyrazol-3-carboxamides:

A sodium enolate of formula (III) is heated with hydrazine sulfate in an aqueous solution of sodium hydroxide for 2 to 3 hours, and the reaction product is brought into reaction with thionyl chloride to provide a 5-alkylpyrazol-3-carboxylic acid chloride. The acid chloride is then reacted with an amine derivative of the general formula, $NH_2-R^5$, wherein $R^5$ represents a group $-CH_2CH_2OH$ or a group $-CH_2\underset{O}{\underset{\|}{C}}-R^6$, wherein $R^6$ is a hydroxyl group or a lower alkoxy group, or a hydrochloride thereof, in dichloroethane in the presence of triethylamine to provide the desired compound.

(3) Synthesis of 3-hydroxymethyl-5-alkylpyrazoles:

A sodium enolate of formula (III) is reacted with hydrazine hydrate in a mineral acid with heating for 4 to 10 hours under reflux to provide an ethyl-5-alkyl-pyrazole-3-carboxylate, which is reduced with lithium aluminum hydride in anhydrous tetrahydrofuran to provide a 3-hydroxymethyl-5-alkylpyrazole.

(4) Synthesis of 1-alkyl-5-alkylpyrazole-3-carboxylic acids:

A sodium enolate of formula (III) is dissolved in distilled water or methanol together with a monosubstituted hydrazine sulfate of the general formula (IV):

$$R^2\text{-NH.NH}_2\text{.H}_2\text{SO}_4 \qquad \text{(IV)}$$

wherein $R^2$ represents alkyl of 1 to 3 carbon atoms, and to the thus obtained solution an aqueous solution of sodium hydroxide is added, and then the mixture is heated for 1 to 2 hours to provide the desired product.

(5) Synthesis of 1-phenyl-5-n-alkylpyrazole-3-carboxylic acids:

A sodium enolate of formula (III) is dissolved in distilled water or methanol together with phenylhydrazine sulfate, and to the thus obtained solution an aqueous solution of sodium hydroxide is added, and then the mixture is heated for 1 to 2 hours to provide the desired products.

The synthesis of various pyrazole derivatives of formula (I) and their physical- and chemical properties will be described below in Examples 1 to 25. Before that an explanation will be provided of the pharmacological properties of the present pyrazole derivatives:

Pharmacological tests were carried out on each compound of the present pyrazole derivatives using

nicotinic acid for comparison.

(i) Pharmacological test against hypertriglyceridemia:

Seven groups of male SD rats, each group consisting of 6 animals, were used as test animals and one group consisting of 12 animals was used as a control. The test groups were made hypertriglyceridemic by administering aqueous 10% by weight solution of fructose for 2 days, while the animals of the control group were given tap water. The administration to the test groups of six pyrazole derivatives and of nicotinic acid was as follows:

Each pyrazole derivative and nicotinic acid was separately dissolved or dispersed in an aqueous 1% solution of carboxymethylcellulose (CMC) at a predetermined concentration, and the thus prepared solution or dispersion was orally administered in three doses, each dose having a volume of 0.5 ml/100 g body weight, a first dose just after the commencement of the test, a second dose 24 hours after the first dose, and a third dose 18 hours after the second dose. The total dosage of each compound was such that each animal received 300 mg/kg of each compound as free carboxylic acid. The rats of the control group were given the aqueous 1% solution of CMC only, in three doses at the same intervals as the test groups. All the groups were fed with the same diet during the test period.

After 48 hours of the commencement of the test, a blood sample was collected from each animal by amputation of the carotid artery, and serum was separated from the blood sample and frozen to be kept until lipid analysis.

<u>Lipid analysis</u>:

Serum triglyceride was determined by Acetylacetone Method, and the reduction percentage of lipid was calculated on the basis of the following formula:

$$\left(1 - \frac{\text{amount of triglyceride in serum of test animal}}{\text{amount of triglyceride in serum of control}}\right) \times 100$$

= the reduction percentage of lipid wherein the reduction percentage is the average for the test animals in each group.

Analysis for serum cholesterol:

The content of serum cholesterol was determined by an enzymatic method using cholesteroloxidase, and the reduction percentage of cholesterol was calculated according to the following formula:

$$\left(1 - \frac{\text{amount of cholesterol in serum of test animal}}{\text{amount of cholesterol in serum of control}}\right) \times 100$$

= the reduction percentage of cholesterol

The results of the analyses are shown in Table 1.

TABLE 1

| Compound | Total Dose (mg/kg) | Reduction Percentage | |
|---|---|---|---|
| | | Triglyceride | Cholesterol |
| 5-n-tridecylpyrazole-3-carboxylic acid | 300 | 76.4 | 59.8 |
| 5-n-pentadecylpyrazole-3-carboxylic acid | 300 | 62.8 | 32.1 |
| 5-n-heptadecylpyrazole-3-carboxylic acid | 300 | 30.5 | 24.5 |
| Ethyl 5-n-tridecyl-pyrazole-3-carboxylate | 329 | 88.0 | 65.1 |
| Ethyl 5-n-pentadecyl-pyrazole-3-carboxylate | 326 | 67.8 | 34.1 |
| Ethyl 5-n-heptadecyl-pyrazole-3-carboxylate | 324 | 35.2 | 32.9 |
| 3-hydroxymethyl-5-tridecylpyrazole | 300 | 78.2 | 55.8 |
| 3-n-ethoxylcarbonyl-methylcarbamoyl-5-tridecylpyrazole | 300 | 81.4 | 34.8 |
| 1-methyl-5-n-tridecyl-pyrazole-3-carboxylic acid | 300 | 75.9 | 61.2 |
| Nicotinic acid | 300 | 24.1 | 16.4 |

(ii) Pharmacological test against hypercholesterolemia:

Three groups of male SD rats each weighing about 150g, each group consisting of five rats, were used as test animals and one group consisting of 10 rats was used as control. All animals were fed a high cholesterol diet consisted of 1% cholesterol, 0.2% sodium cholate, 5% olive oil and commercial diet up to total 100% for 7 days. The test compounds of the present invention and clofibrate, as a medicine for comparison, were respectively dissolved or dispersed in an aqueous 1% solution of CMC so that the volume of the solution or dispersion administered each time to an animal was 0.5 ml/100g body weight, and the solution or dispersion was orally administered so that the amount of administration each time of each pyrazole derivative or of clofibrate was 200 mg/kg body weight. Administration was carried out once a day for 7 consecutive days. Water was taken ad lib. After 20 hours of fasting from the last administration, a blood sample was taken from each animal by amputation of the carotid artery, and the serum separated was frozen until the analysis of cholesterol shown as follows:

Cholesterol analysis

The content of serum cholesterol was determined by the above-mentioned method. The beta-lipoprotein-cholesterol was determined by "heparin-calcium precipitation method". The reduction percentage of cholesterol in serum and beta-lipoprotein were calculated in the same manner as above. The results are shown in Table 2.

TABLE 2

| Compound | Dose (mg/kg/day) | Reduction Percentage: Cholesterol | Reduction Percentage: Cholesterol in beta-lipoprotein |
|---|---|---|---|
| 5-n-tridecylpyrazole-3-carboxylic acid | 200 | 45.2 | 54.3 |
| 3-hydroxymethyl-5-n-tridecylpyrazole | 200 | 52.7 | 25.2 |
| 3-n-ethoxycarbonylmethylcarbamoyl-5-n-tridecylpyrazole | 200 | 48.6 | 32.7 |
| 1-methyl-5-n-tridecylpyrazole-3-carboxylic acid | 200 | 60.1 | 57.3 |
| Clofibrate | 200 | - 3.5 | - 2.0 |

As seen from Tables 1 and 2, the present pyrazole derivatives have an excellent antilipemic activity superior to that of nicotinic acid and clofibrate, known antilipemic agents.

The acute toxicity of the present pyrazole derivatives to mammals was determined by the following method. The results showed that each of the present pyrazole derivatives had a $LD_{50}$ value over 5.0 g/kg body weight significantly larger than that of nicotinic acid, 4.5 g/kg.

Test method for acute toxicity:

Male ICR mice of body weight 21 to 25 g were

divided into several groups each consisting of 8 animals and were fasted for 17 to 18 hours and then orally given a pyrazole derivative or nicotinic acid dissolved or dispersed in an aqueous 1% CMC solution at a rate of 0.2 ml/10 g body weight. The administered amount of each compound was 2.5 g/kg body weight or 5.0 g/kg, and from the mortalities at both levels, $LD_{50}$ was roughly calculated and compared with $LD_{50}$ of nicotinic acid.

The following description is an explanation of formulating the present pyrazole derivatives into pharmaceutical compositions. In general, the present pyrazole derivative can be formulated into a pharmaceutical composition of dose unit form by known techniques. Since a present pyrazole derivative is generally administered orally, it is preferably formulated into tablets or capsules. When formulating a present pyrazole derivative into the dose unit form, vehicles, disintegrating agents, binding agents and lubricating agents which are used widely in the pharmaceutical field may be used.

The dose level of the present pyrazole derivative naturally depends upon the degree of hyperlipemia, but, in general, a suitable daily oral dose will be 0.2 to 3.0 g/60 kg of body weight, preferably 0.5 to 1.5 g/60 kg body weight.

Examples 1 to 25 which follow provide a more detailed explanation of the synthesis of the pyrazole derivatives, while Examples 26 and 27 describe their formulation.

EXAMPLE 1

Synthesis of ethyl 5-n-tridecylpyrazole-3-carboxylate: 10.4 g (0.45 mol) of metallic sodium was dissolved in 500 ml of anhydrous ethanol and then a liquid mixture consisting of 100 g (0.44 mol) of methyl n-tridecyl ketone and 64 g (0.44 mol) of diethyl oxalate was slowly dropped into the solution with stirring. After the addition, the mixture was heated for 5 hours at a temperature of 60°C under stirring. Then, the reaction mixture was cooled by iced water, and the resulting yellow precipitate was collected by filtration and recrystallized from ethanol to provide 103.3 g (0.1 mol) of sodium 1-ethoxycarbonyl-3-oxo-1-hexadecenolate, $CH_3(CH_2)_{12}\underset{\underset{O}{\|}}{C}-CH=\underset{\underset{ONa}{|}}{C}-CO_2C_2H_5$, as pale yellow crystals melting at 68 - 70°C at a yield of 67.5%.

To a solution of 34.8 g (0.1 mol) of the sodium 1-ethoxycarbonyl-3-oxo-1-hexadecenolate in 23 ml of glacial acetic acid 5.5 g (0.11 mol) of hydrazine hydrate was slowly dropped under cooling with iced water. After heating the mixture for 8 hours under reflux, the reaction mixture was cooled and poured into iced water, then neutralized with a saturated aqueous solution of sodium hydrogen carbonate and extracted with benzene. The separated benzene layer was washed with a saturated aqueous solution of sodium hydrogen carbonate and then dried over anhydrous sodium sulfate. Solvent was evaporated and the residue was recrystallized from ethyl ether to provide 22.6 g of ethyl 5-n-tridecylpyrazole-3-carboxylate as colourless needle-like crystals melting at 45-46°C at a yield of 70.0%.

Elementary analysis:

found : 70.76% C; 10.66% H; 8.67% N calcd. as $C_{19}H_{34}O_2N_2$ : 70.76% C; 10.63% H; 8.69% N.

Infrafred absorption spectrum (hereinafter referred to

as IR):
(nujol method)
$\nu_{max}^{nujol}$ 3200 - 3100 $cm^{-1}$ ($\nu$NH) and 1715 $cm^{-1}$ ($\nu$C=O).
Mass spectrum (hereinafter referred to as Mass):
m/e 322 ($M^+$)
Nuclear magnetic resonance spectrum (hereinafter referred to as NMR): (in $CDCl_3$) $\delta$ :
0.87 [3 H, t, J=$6H_z$, $\underline{CH_3}\ CH_2)_{12}$]
1.2 - 1.5 [23H, $\underline{CH_3(CH_2)_{10}}(CH_2)_2$, $CH_3CH_2OCO$]
1.7 (2H, m, $C_{11}H_{23}\underline{CH_2}CH_2$)
2.70 (2 H, t, J=8Hz, $C_{12}H_{25}\underline{CH_{12}}$)
4.38 (2 H, q, J=7Hz, $CH_3\underline{CH_2}OCO$)
6.60 (1 H, s, H in the pyrazole ring)
11.95 (1H, br, NH which disappears by treating with $D_2O$).

EXAMPLE 2

Synthesis of 5-n-tridecylpyrazole-3-carboxylic acid:

A solution of 10 g (0.031 mol) of ethyl 5-n-tridecylpyrazole-3-carboxylate in 50 ml of a 5% methanolic solution of sodium hydroxide was heated under reflux for 5 hours. After adding distilled water, methanol was evaporated under a reduced pressure and the residue was made acidic (pH of about 2) by adding concentrated hydrochloric acid. The separated precipitate was collected by filtration and recrystallized from ethanol to provide 7.2 g of 5-n-tridecylpyrazole-3-carboxylic acid as needle-like crystals melting at 141 - 143°C, a yield of 79.2%.
Elementary analysis:
found : 65.31% C; 9.47% H; 9.58% N.
calcd. as $C_{17}H_{30}O_2N_2H_2O$: 65.35% C; 10.32% H; 8.97% N.
IR (KBr-method):

$\nu_{max}^{KBr}$ 3240 $cm^{-1}$ (br. $\nu$CH, NH), 3000 - 2300 $cm^{-1}$ (br.$\nu NH_2$+ and $CO_2H$) and 1680 $cm^{-1}$ ($\nu$C=O)

Mass : m/e : 294 ($M^+$)

NMR (in $d_6$-DMSO) δ :

0.85 (3H, t, J=6 Hz, $\underline{CH_3}(CH_2)_{12}$)

1.2 - 1.5 (20H, br. s, $CH_3\underline{(CH_2)_{10}}(CH_2)_2$)

1.60 (2H, m, $CH_3(CH_2)_{10}\underline{CH_2}CH_2$)

2.59 (2H, t, J=8 Hz, $CH_3(CH_2)_{11}\underline{CH_2}$)

4.72 (2H, br. NH and $CO_2H$, which disappears by treatment with $D_2O$)

6.35 (1H, s, H in pyrazole ring)

EXAMPLE 3

Synthesis of 5-n-tridecylpyrazole-3-carboxylic acid:

After dissolving 20 g (0.0575 mol) of sodium 1-ethoxycarboxyl-3-oxo-1-hexadecenolate and 8.2 g (0.0633 mol) of hydrazine sulfate in 40 ml of methanol with agitation under warming, 11.5 ml of an aqueous 33% solution of sodium hydroxide were added dropwise into the solution. After the addition, the mixture was heated for 2 hours under reflux and methanol was evaporated after adding distilled water. After making the pH of the residue nearly to 2 with concentrated hydrochloric acid, water was completely removed by distillation under a reduced pressure, and the residue was extracted with 500 ml of hot acetone. After drying the acetonic extract and removing the solvent, the residue was recrystallized from ethanol to provide 5-n-tridecylpyrazole-3-carboxylic acid as colourless in a yield of 67.4%.

The physical properties of the product were identical to those of the product prepared in Example 2.

EXAMPLE 4

Synthesis of ethyl 5-n-pentadecylpyrazole-3-carboxylate:

5.5 g of hydrazine hydrate was dropped slowly into a solution of 40.0 g (0.106 mol) of sodium 1-ethoxycarbonyl-3-oxo-1-octadecenolate in 20 ml of glacial acetic acid under cooling with iced water. After heating the mixture for 5 hours under reflux, the reaction mixture was cooled and poured into iced water and then neutralized with a saturated aqueous solution of sodium hydrogen carbonate and extracted with benzene. After washing the benzene layer with a saturated aqueous solution of sodium hydrogen carbonate and drying over anhydrous sodium sulfate, the solvent was removed by distillation under a reduced pressure. The residue was recrystallized from ethanol to provide 13.4 g of ethyl 5-n-pentadecylpyrazole-3-carboxylate as colourless plate-like crystals melting at 53 to 54°C in a yield of 36.4%.

Elementary analysis:

found : 72.03% C; 11.25% H; 7.74% N.

calcd. as $C_{21}H_{38}O_2N_2$: 71.95% C; 10.93% H; 7.99% N.

IR :

$\nu_{max}^{nujol}$ 3200 - 3100 $cm^{-1}$ ($\nu$NH) and 1718 $cm^{-1}$ ($\nu$C = O)

Mass : m/e 350 ($M^+$)

NMR (in $CDCl_3$) δ :

0.92 (3H, t, J=6 Hz, $\underline{CH_3}(CH_2)_{14}$)

1.2 - 1.6 (27H, $CH_3\underline{(CH_2)_{12}}(CH_2)_2$ and $\underline{CH_3}CH_2OCO$)

1.7 (2H, m, $C_{13}H_{27}\underline{CH_2}CH_2$)

2.90 (2H, t, J=8 Hz, $C_{14}H_{29}\underline{CH_2}$)

4.72 (2H, q, J=8 Hz, $CH_3\underline{CH_2}OCO$)

7.05 (1H, s, H in the pyrazole ring)

12.35 (1H, br, NH which disappears by treatment with $D_2O$)

EXAMPLE 5

Synthesis of 5-n-pentadecylpyrazole-3-carboxylic acid:

A solution of 13.0 g (0.037 mol) of ethyl 5-n-pentadecylpyrazole-3-carboxylate in 100 ml of an aqueous 5% ethanolic solution of sodium hydroxide was heated for 7 hours under reflux. After adding distilled water, ethanol was evaporated by distillation under a reduced pressure. The residue was made acidic (pH of nearly 2) with concentrated hydrochloric acid, and then the deposited pale yellow needle-like crystals were collected by filtration and recrystallized from ethanol to provide 5-n-pentadecylpyrazole-3-carboxylic acid as colourless needle-like crystals melting at 133 - 135°C in an amount of 6.7 g corresponding to a yield of 56.0%.

Elementary analysis:

found: 67.34% C; 10.57% H; 8.08% N.

calcd. as $C_{19}H_{34}O_2N_2 \cdot H_2O$ :

67.01% C; 10.66% H; 8.23% N.

IR:

$\nu_{max}^{KBr}$ 3240 $cm^{-1}$ (br, $\nu$NH, OH), 3000 - 2300 $cm^{-1}$ (br, $\nu NH_2$+ and COOH) and 1710 $cm^{-1}$ ($\nu$C = O)

Mass: m/e 322 ($M^+$)

NMR (in $d_6$-DMSO) $\delta$ :

0.86 (3H, t, J=7 Hz, $\underline{CH_3}(CH_2)_{14}$)

BAD ORIGINAL

1.2 - 1.5 (24H, br. s, $CH_3(\underline{CH_2})_{\underline{12}}(CH_2)_2$)

1.60 (2H, m, $C_{13}H_{27}\underline{CH_2}CH_2$)

2.64 (2H, t, J=8 Hz, $C_{14}H_{29}\underline{CH_2}$)

6.50 (1H, s, H in the pyrazole ring)

10.60 (2H, br, NH and $CO_2H$ which disappears by treatment with $D_2O$.)

EXAMPLE 6

Synthesis of ethyl 5-n-heptadecylpyrazole-3-carboxylate:

3.2g (0.066 mol) of hydrazine hydrate was dropped slowly into a solution of 24.2g (0.06 mol) of sodium 1-ethoxycarbonyl-3-oxo-1-eicosenolate in 13 ml of glacial acetic acid under cooling with iced water. After heating the mixture for 2 hours under reflux, the reaction mixture was poured into iced water and then was neutralized by a saturated aqueous solution of sodium hydrogen carbonate and extracted with benzene. The benzene layer was washed with a saturated aqueous solution of sodium hydrogen carbonate and dried over anhydrous sodium sulfate. After removing the solvent by distillation under a reduced pressure, the residue was recrystallized from ethyl ether to provide ethyl 5-n-heptadecylpyrazole-3-carboxylate as colourless needle-like crystals melting at 56 - 57°C in an amount of 13.7g corresponding to a yield of 65.2%.

Elementary analysis:

found : 72.78% C; 11.25% H; 9.58% of N.

calcd. as $C_{23}H_{42}O_2N_2$ : 72.96% C; 11.18% H; 8.97% N.

IR:

$\nu_{max}^{nujol}$ 3200 - 3100 $cm^{-1}$ ($\nu$NH) and 1715 $cm^{-1}$ ($\nu$C = O)

Mass: m/e 378 ($M^+$)

NMR (in $d_6$-$CDCl_3$) δ :

0.90 (3H, t, J = 6 Hz, $\underline{CH_3}(CH_2)_{16}$)

1.2 - 1.5 (31H, $CH_3\underline{(CH_2)_{14}}(CH_2)_2$ and $\underline{CH_3}CH_2OCO$)

1.7 (2H, m, $C_{15}H_{31}\underline{CH_2}CH_2$)

2.55 (2H, t, J = 8 Hz, $C_{16}H_{33}\underline{CH_2}$)

4.30 (2H, q, J = 7 Hz, $CH_3\underline{CH_2}OCO$)

5.95 (1H, br, NH which disappears by treatment with $D_2O$)

6.45 (1H, s, H in the pyrazole ring).

EXAMPLE 7

Synthesis of 5-n-heptadecylpyrazole-3-carboxylic acid:

By the same procedures as in Example 2 ethyl 5-n-heptadecylpyrazole-3-carboxylate (0.026 mol) was hydrolyzed to the corresponding free acid. The crude pale yellow precipitate was recrystallized from ethanol to provide 6.8g of colourless needle-like crystals melting at 125 - 127°C. The yield was 69.5%.

Elementary anlaysis:

found : 69.02% C; 10.55% H; 7.89% N

calcd. as $C_{21}H_{38}O_2N_2 \cdot H_2O$ : 68.43% C; 10.94% H; 7.60% N.

IR:

$\nu_{max}^{KBr}$ at 3240 $cm^{-1}$ (br. νNH, OH), 3000 - 2300 $cm^{-1}$ (br. $\nu NH_2^+$ and COOH) and 1680 $cm^{-1}$ (νC = O).

Mass: m/e 350 ($M^+$).

NMR (in $d_6$-DMSO) δ:

0.85 (3H, t, J = 7 Hz, $\underline{CH_3}(CH_2)_{16}$)

1.2 - 1.7 (30H, br. s, $CH_3\underline{(CH_2)_{15}}CH_2$)

2.65 (2H, t, J = 7 Hz, $C_{16}H_{33}\underline{CH_2}$)

6.48 (1H, s, H in the pyrazole ring)

7.60 (2H, br. NH and $CO_2H$ which disappear by treatment with $D_2O$)

EXAMPLE 8

Synthesis of 3-N-2-hydroxyethylcarbamoyl-5-n-nonylpyrazole:

An excess of thionyl chloride was added to 2.5g (0.011 mol) of 5-n-nonylpyrazole-3-carboxylic acid and after heating the mixture for 2 hours under agitation at 42 to 43°C, the reaction mixture was condensed to solid under a reduced pressure to obtain 5-n-nonylpyrazole-3-carboxylic acid chloride as yellow crystals.

A solution of 0.013 mol of et olamine and 1.1g (0.011 mol) of triethylamine in 70 ml of dichloroethane was slowly dropped into the acid chloride under cooling with iced water. After agitating the mixture for 8 hours at room temperature, the thus deposited triethylamine hydrochloride was removed by filtration, and iced water was added to the filtrate. The organic phase was separated and dried over anhydrous sodium sulfate, and the solvent therein was distilled off to obtain a residue, which was recrystallized from acetone to provide the desired product as colourless needle-like crystals melting at 48 - 50°C in an amount of 1.6g corresponding to a yield of 51.8%.

Elementary analysis:

found: 61.85% C; 9.47% H and 14.47% N

calcd. as $C_{15}H_{27}O_2N_3 \cdot 1/2\ H_2O$ :

62.04% C; 9.72% H and 14.47% N.

IR:

$\nu^{nujol}_{max}$ 3400 - 3000 $cm^{-1}$ ($\nu$NH, OH) and 1625 and 1550 $cm^{-1}$ ($\nu$ amide).

Mass : m/e 281 ($M^+$).

NMR (in $CDCl_3$) δ :

0.86 (3H, t, J = 6 Hz, $\underline{CH_3}(CH_2)_8$)

1.2 - 1.5 (12H, br. s, $CH_3\underline{(CH_2)_6}(CH_2)_2$)

1.6 (2H, m, $C_7H_{15}\underline{CH_2}CH_2$)

2.62 (2H, t, J = 7 Hz, $C_8H_{17}\underline{CH_2}$)

3.38 and 3.55 (2H each, m, $OCNH\underline{CH_2}CH_2OH$)

5.4 (1H, s, H in the pyrazole ring)

8.10 (1H, t-like, $OCN\underline{H}CH_2$)

<u>EXAMPLE 9</u>

Synthesis of 3-N-2'-hydroxyethylcarbamoyl-5-n-undecylpyrazole:

By the same procedures as in Example 8, 3.0g (0.11 mol) of 5-n-undecylpyrazole-3-carboxylic acid was transformed to the corresponding amide of hydroxyethylamine. The crude produce was recrystallized from acetone to provide the desired product as colourless needle-like crystals melting at 63 - 65°C in an amount of 1.3g corresponding to a yield of 38.3%.

Elementary analysis:

found : 66.09% C; 10.19% H and 13.28% N

calcd. as $C_{17}H_{31}O_2N_3$ :

65.95% C; 10.10% H and 13.58% N.

IR:

$\nu^{nujol}_{max}$ 3400 - 3000 $cm^{-1}$ (νNH and OH) and 1630 and 1560 $cm^{-1}$ (ν amide).

Mass: m/e 309 ($M^+$)

NMR (in $CDCl_3$) δ :

0.86 (3H, t, J = 6 Hz, $\underline{CH_3}(CH_2)_{10}$)

1.2 - 1.5 (16H, br. s, $CH_3\underline{(CH_2)_8}CH_2CH_2$)

1.6 (2H, m, $C_9H_{19}\underline{CH_2}CH_2$)

2.60 (2H, t, J = 7 Hz, $C_{10}H_{21}\underline{CH_2}$)

3.55 and 3.75 (2H each, m, $OCNH\underline{CH_2}-\underline{CH_2}OH$)

6.50 (1H, s, H in the pyrazole ring)

7.20 (2H, br. NH and OH which disappears by treatment with $D_2O$)

8.10 (1H, br. NH which disappears by treatment with $D_2O$)

EXAMPLE 10

Synthesis of 3-N-2-hydroxyethylcarbamoyl-5-n-tridecylpyrazole:

By the same procedures as in Example 8, 4.0g (0.014 mol) of 5-n-tridecylpyrazole-3-carboxylic acid was transformed to the corresponding amide of hydroxyethylamine. The crude product was recrystallized from acetone to provide colourless needle-like crystals of the desired product melting at 78 - 79°C in an amount of 2.1g corresponding to a yield of 45.1%.

Elementary analysis:

found : 67.51% of C, 10.16% of H and 12.20% of N

calcd. as $C_{19}H_{35}O_2N_3$ : 67.61% of C, 10.45% of H and 12.45% of N.

IR:

$\nu_{max}^{nujol}$ 3400 - 3000 $cm^{-1}$ (νNH and OH) and 1635 and 1555 $cm^{-1}$ (ν amide).

Mass : m/e 337 ($M^+$).

NMR (in $CDCl_3$) δ:

0.87 (3H, t, J = 6 Hz, $\underline{CH_3}(CH_2)_{12}$)

1.2 - 1.5 (2H, br. s, $CH_3\underline{(CH_2)_{10}}(CH_2)_2$)

1.6 (2H, m, $C_{11}H_{23}\underline{CH_2}-CH_2$)

2.60 (2H, t, J = 7 Hz, $C_{12}H_{25}$ - $\underline{CH_2}$)

3.55 and 3.80 (2H each, m, CONH-$\underline{CH_2}$-$\underline{CH_2}$-OH)

5.0 (2H, br. NH and OH which disappears by treatment with $D_2O$)

6.38 (1H, s, H in the pyrazole ring)

7.84 (1H, br. NH which disappears by treatment with $D_2O$)

EXAMPLE 11

Synthesis of 3-N-ethoxycarbonylmethylcarbamoyl-5-n-nonylpyrazole:

An excess of thionyl chloride was added to 3.1g (0.031 mol) of 5-n-nonylpyrazole-3-carboxylic acid, and after heating the mixture for 3 hours at 42 to 43°C with agitation, the reaction mixture was condensed to a solid under a reduced pressure, and the thus formed 5-n-nonylpyrazole-3-carboxylic acid chloride was dissolved in 25 ml of dichloroethane. This solution was slowly added dropwise to a mixture of 2.0g (0.014 mol) of ethyl ester of glycine hydrochloride, 10 ml of triethylamine and 25 ml of dichloroethane with agitation at room temperature. After the addition, the mixture was heated at 60 to 62°C for 10 hours with agitation. After cooling the reaction system with ice and the thus deposited triethylamine hydrochloride was removed by filtration, distilled water was added to the filtrate, and organic layer was concentrated under reduced pressure, after drying over anhydrous sodium sulfate. Recrystallization of the residue from acetone gave colourless crystals of the desired product, melting at 34 to 35°C in amount of 1.9g corresponding to the yield of 46.4%.

Elementary analysis:

found : 62.70% C; 9.35% H and 12.73% N

calcd. as $C_{17}H_{29}O_3N_3$ :

63.13% C; 9.04% H and 12.99 N.

IR:

$\nu_{max}^{nujol}$ 3500 - 3100 $cm^{-1}$($\nu$NH and OH), 1755 $cm^{-1}$($\nu$ ester) and 1660 and 1555 $cm^{-1}$ ($\nu$ amide)

Mass: m/e 323 ($M^+$).

NMR (in $CDCl_3$) $\delta$ :

0.85 (3H, t, J = 6 Hz, $\underline{CH_3}(CH_2)_8$)

1.2 - 1.5 (15H, $CH_3\underline{(CH_2)_6}(CH_2)_2$ and $\underline{CH_3}CH_2OCO$)

1.6 (2H, m, $C_7H_{15}\underline{CH_2}-CH_3$)

2.62 (2H, t, J = 8 Hz, $C_8H_{17}\underline{CH_2}$)

4.15 - 4.40 (4H, m, CONH-$\underline{CH_2}$-$CO_2$-$\underline{CH_2}$-$CH_3$)

6.52 (1H, s, H in the pyrazole ring)

7.6 (1H, br, NH in the ring, which disappear by treatment with $D_2O$)

7.95 (1H, t-like, NH which disappear by the treatment with $D_2O$).

EXAMPLE 12

Synthesis of 3-N-ethoxycarbonylmethylcarbamoyl-5-n-undecylpyrazole:

By the same procedures as in Example 11, 3.5 (0.013 mol) of 5-n-undecylpyrazole-3-carboxylic acid, was transformed to the corresponding amide of glycine. The crude product was recrystallized from acetone to provide the desired product as colourless crystals melting at 48 to 49°C in an amount of 2.0g corresponding to a yield of 43.8%.

Elementary analysis:

found : 64.37% C; 9.38% H and 11.25% N

calcd. as $C_{19}H_{33}O_3N_3$ :

64.92% C; 9.46% H and 11.96% N.

IR:

$\nu_{max}^{nujol}$ 3500 - 3100 $cm^{-1}$ ($\nu$NH and OH), 1750 $cm^{-1}$ (ester) 1660 and 1560 ($\nu$ amide)

Mass : m/e 351 ($M^+$).

NMR (in $CDCl_3$) $\delta$ :

0.89 (3H, t, J = 6 Hz, $\underline{CH_3}(CH_2)_{10}$)

1.2 - 1.5 (19H, $CH_3\underline{(CH_2)_8}(CH_2)_2$ and $\underline{CH_3}CH_2OCO$)

1.6 (2H, m, $C_9H_{19}\underline{CH_2}-CH_2$)

2.67 (2H, t, J = 7 Hz, $C_{10}H_{21}$ - $\underline{CH_2}$)

4.15 - 4.40 (4H, m, CONH $\underline{CH_2}CO_2$ $\underline{CH_2}CH_3$)

6.62 (1H, s, H in the pyrazole ring)

8.20 (1H, t-like, NH which disappear by treatment with $D_2O$)

11.10 (1H, br. NH in the ring, disappearing by treatment with $D_2O$)

EXAMPLE 13

Synthesis of 3-N-ethoxycarbonylmethylcarbamoyl-5-n-tridecylpyrazole:

By the same procedures as in Example 11, 2.9g (0.01 mol) of 5-n-tridecylpyrazole-3-carboxylic acid, was transformed to the corresponding amide of glycine. The crude product was recrystallized from acetone to provide the desired product as colourless needle-like crystals melting at 54 to 55°C in an amount of 1.8g corresponding to a yield of 47.4%.

Elementary analysis:

found: 66.30% C; 10.03% H and 10.81% N

calcd. as $C_{21}H_{37}O_3N_3$ :

66.45 of C, 9.83% of H and 11.07% of N.

IR:

$\nu_{max}^{nujol}$ 3400 - 3100 $cm^{-1}$($\nu$NH,OH), 1730 $cm^{-1}$($\nu$ ester) and 1640 and 1560 $cm^{-1}$ ($\nu$ amide)

Mass: m/e 379 ($M^+$).

NMR (in $CDCl_3$) δ :

0.89 (3H, t, J = 6 Hz, $CH_3(CH_2)_{12}$)

1.2 - 1.5 (23H, $CH_3\underline{(CH_2)_{10}}(CH_2)_2$ and $\underline{CH_3}CH_2OCO$)

1.6 (2H, m, $C_{11}H_{23}-\underline{CH_2}-CH_2$)

2.63 (2H, t, J = 7 Hz, $C_{12}H_{25}\underline{CH_2}$)

4.0 - 4.4 (4H, m, CONH-$\underline{CH_2}$-$CO_2$-$\underline{CH_2}CH_3$)

6.55 (1H, s, H in the pyrazole ring)

8.00 and 9.35 (1H each, br. NH X 2 which disappears by treatment with $D_2O$)

EXAMPLE 14

Synthesis of 3-hydroxymethyl-5-n-tridecylpyrazole:

A solution of 11.0g (0.034 mol) of ethyl 5-n-tridecylpyrazole-3-carboxylate in 100 ml of anhydrous tetrahydrofuran, a suspension of 1.6g (0.042 mol) of lithium aluminum hydride in 150 ml of anhydrous tetrahydrofuran was added dropwise with stirring under cooling with ice. After stirring for 12 hours at room temperature, water-containing tetrahydrofuran was added to the reaction mixture. The deposited white precipitate was removed by filtration, and the filtrate was condensed to a solid and the residue recrystallized from ethyl ether to provide the desired product as colourless needle-like crystals melting at 64 to 66°C in an amount of 7.8g corresponding to a yield of 81.7%.

Elementary analysis:

found: 72.85% C; 11.60% H and 10.01% N

calcd. as $C_{17}H_{32}ON_2$ : 72.80% C; 11.50% H and 9.99% N.

IR:

$\nu_{max}^{nujol}$ 3300 - 3100 $cm^{-1}$ (ν NH and OH), and 1570 (ν pyrazole ring).

Mass : m/e 280 ($M^+$).

NMR (in $CDCl_3$) δ :

0.89 (3H, t, J = 6 Hz, $\underline{CH_3}(CH_2)_{12}$)

1.2 - 1.5 (2H, br. s, $CH_3\underline{(CH_2)_{10}}(CH_2)_2$)

1.6 (2H, m, $C_{11}H_{23}$-$\underline{CH_2}$-$CH_2$)

2.56 (2H, t, J = 8 Hz, $C_{12}H_{25}$ - $\underline{CH_2}$)

4.60 (2H, s, $\underline{CH_2}OH$)

5.94 (1H, s, H in the pyrazole ring)

6.37 (2H, br. s, NH and OH disappearing by treatment with $D_2O$).

EXAMPLE 15

Synthesis of 3-hydroxymethyl-5-n-heptylpyrazole:

In the same manner as in Example 14, 11.5g of ethyl 5-n-heptylpyrazole-3-carboxylate was reduced to the corresponding alcohol. The crude product was recrystallized from ethanol and n-hexane to provide the desired product as white plate-like crystals melting at 40 to 41°C in an amount of 7.5g corresponding to a yield of 76.9% of theoretical.

Elementary analysis:

found : 67.03% C; 10.18% H and 13.99% N

calcd. as $C_{11}H_{20}ON_2$ :

67.30% C; 10.20% H and 14.27% N.

IR:

$\nu_{max}^{nujol}$ 3300 - 3100 $cm^{-1}$ (ν NH and OH) and 1580 (ν pyrazole ring)

Mass: m/e 196 ($M^+$).

NMR (in $CDCl_3$) δ :

0.85 (3H, t, J = 6 Hz, $\underline{CH_3}(CH_2)_6$)

1.2 - 1.5 (8H, br. s, $CH_3\underline{(CH_2)}$ $(CH_2)_2$)

1.6 (2H, m, $C_5H_{11}$-$\underline{CH_2}$-$CH_2$)

2.50 (2H, t, J = 8 Hz, $C_6H_{13}$ - $\underline{CH_2}$)

4.53 (2H, s, $\underline{CH_2}$-OH)

5.83 (1H, s, H in the pyrazole ring)

8.48 (2H, br. s, NH and OH disappearing by treatment with $D_2O$).

EXAMPLE 16

Synthesis of 3-hydroxymethyl-5-n-nonyl-pyrazole:

In the same manner as in Example 14, 14.5 g of -5-n-nonylpyrazole-3-carboxylate was reduced to the corresponding alcohol. The crude product was recrystallized from ethyl ether to provide the desired product as colourless needle-like crystals melting at 52 to 53°C in an amount of 10.6 g corresponding to a yield of 85.2%.

Elementary analysis:

found : 69.70% C; 10.68% H and 12.74% N

calcd. as $C_{13}H_{24}ON_2$ :

69.60% C; 10.78% H and 12.49% N.

IR :

$\nu^{nujol}_{max}$ 3300 - 3100 $cm^{-1}$ ($\nu$ NH, OH) and 1580 $cm^{-1}$ ($\nu$ pyrazole group).

Mass: m/e 224 ($M^+$).

NMR (in $CDCl_3$) δ :

0.89 (3H, t, J = 6 Hz, $\underline{CH_3}(CH_2)_8$)

1.2 - 1.5 (12H, br. s, $CH_3\underline{(CH_2)_6}(CH_2)_2$)

1.6 (2H, m, $C_7H_{15}-\underline{CH_2}-CH_2$)

2.54 (2H, t, J = 8 Hz, $C_8H_{17} - \underline{CH_2}$)

4.58 (2H, s, $\underline{CH_2}-OH$)

5.91 (1H, s, H in the pyrazole ring)

8.00 (2H, br. s, NH and OH disappearing by treatment with $D_2O$)

EXAMPLE 17

Synthesis of 3-hydroxymethyl-5-n-undecyl-pyrazole:

In the same manner as in Example 14, 24.2 g. (0.034 mol) of ethyl 5-n-undecylpyrazole-3-carboxylate, was reduced to the corresponding alcohol. The crude

product was recrystallized from ethyl ether to provide 7.2 g of the desired product as colourless needle-like crystals melting at 56 to 58°C corresponding to a yield of 83.6%.

Elementary analysis:

found : 71.30% C; 11.46% H and 10.95% N

calcd. as $C_{15}H_{28}ON_2$ :
71.33% C; 11.18% H and 11.10% N.

IR:

$\nu_{max}^{nujol}$ 3300 - 3100 $cm^{-1}$ ($\nu$ NH and OH) and 1590 ($\nu$ pyrazole ring).

Mass : m/e 252 ($M^+$).

NMR (in $CDCl_3$) $\delta$ :

0.89 (3H, t, J = 6 Hz, $\underline{CH_3}(CH_2)_{10}$)

1.2 - 1.5 (16H, br. s, $CH_3\underline{(CH_2)_8}$ $(CH_2)_2$)

1.6 (2H, m, $C_9H_{19}$-$\underline{CH_2}$-$CH_2$)

2.55 (2H, t, J = 8 Hz, $C_{10}H_{21}$ - $CH_2$)

4.60 (2H, s, $\underline{CH_2}$-OH)

5.92 (1H, s, H in the pyrazole ring)

8.54 (1H, br. s, NH and OH disappearing by treatment with $D_2O$)

EXAMPLE 18

Synthesis of 1-methyl-5-n-nonylpyrazole-3-carboxylic acid:

20.0 g (0.069 mol) of sodium-1-ethoxycarbonyl-3-oxo-1-dodecenolate and 10.1 g (0.053 mol) of methylhydrazine sulfate were dissolved in 45 ml of distilled water, and then 14 ml of aqueous 33% solution of sodium hydroxide was added dropwise. Then, the mixture was heated for one hour at 80°C with agitation, and after cooling, it was made acidic with 6N sulfuric acid solution and extracted with chloroform. The chloroform

layer was washed with a saturated aqueous solution of sodium chloride, dried over anhydrous sodium sulfate and concentrated under a reduced pressure. By recrystallizing the residue from a mixture of acetone and isopropyl ether, 1-methyl-5-n-nonylpyrazole-3-carboxylic acid was obtained as colourless needle-like crystals melting at 87°C in an amount of 11.3 g corresponding to a yield of 64.7%.

Elementary analysis:

found : 66.90% C; 9.61% H and 11.24% N

calcd. as $C_{14}H_{24}O_2N_2$:

66.63% C; 9.59% H and 11.10% N.

IR :

$\nu_{max}^{nujol}$ 3000 - 2500 $cm^{-1}$ ($\nu$ $CO_2H$) and 1680 $cm^{-1}$ ($\nu$ C = O).

Mass : m/e 252 ($M^+$).

NMR (in $d_6$-DMSO) δ :

0.89 (3H, t, J = 6 Hz, $\underline{CH_3}(CH_2)_8$)

1.2 - 1.6 (12H, br. s, $CH_3\underline{(CH_2)_6}(CH_2)_2$)

1.7 (2H, m, $CH_3\underline{(CH_2)_6}-CH_2-CH_2$)

2.62 (2H, t, J = 8 Hz, $CH_3(CH_2)_7 - \underline{CH_2}$)

3.83 (3H, s, N-$\underline{CH_3}$)

6.50 (1H, s, H in the pyrazole ring)

8.4 (1H, br., $CO_2H$, which disappear by treatment with $D_2O$)

EXAMPLE 19

Synthesis of 1-methyl-5-n-heptylpyrazole-3-carboxylic acid:

In the same manner as in Example 18, 26.4 (0.1 mol) of sodium 1-ethoxycarbonyl-3-oxo-1-decenolate was cyclized to the corresponding N-methylpyrazole derivative. The crude product was recrystallized from ethanol to provide 1-methyl-5-n-heptylpyrazole-3-carboxylic

acid as white powdery crystals melting at 80°C in an amount of 11.7 g corresponding to a yield of 56.5%.

Elementary analysis:

found: 64.50% C; 9.12% H and 12.50% N.

calcd. as $C_{12}H_{20}O_2N_2$ :

64.25% of C, 8.88% of H and 12.49% of N.

IR :

$\nu^{nujol}_{max}$ 3000 -2500 $cm^{-1}$ ($\nu$ $CO_2H$) and 1680 $cm^{-1}$ ($\nu$C=O).

Mass : m/e 224 ($M^+$)

NMR : (in $d_6$-DMSO) $\delta$ :

0.89 (3H, t, J = 6 Hz, $\underline{CH_3}(CH_2)_6$)

1.2 - 1.6 (8H, br. s, $CH_3\underline{(CH_2)_4}(CH_2)_2$)

1.7 (2H, m, $CH_3(CH_2)_4$-$\underline{CH_2}$-$CH_2$)

2.62 (2H, t, J= 8 Hz, $CH_3(CH_2)_5$ - $\underline{CH_2}$)

3.80 (3H, s, N-$\underline{CH_3}$)

6.50 (1H, s, H in the pyrazole ring)

8.4 (1H, br., $CO_2H$ which disappears by treatment with $D_2O$)

EXAMPLE 20

Synthesis of 1-methyl-5-n-undecylpyrazole-3-carboxylic acid:

In the same manner as in Example 18, 20.0 g of sodium 1-ethoxycarbonyl-3-oxo-1-tetradecenolate was cyclized to the corresponding N-methylpyrazole derivative. The crude product was recrystallized from a mixture of acetone and isopropyl ether to provide 1-methyl-5-n-undecylpyrazole-3-carboxylic acid as colourless needle-like crystals melting at 79°C in an amount of 12.6 g corresponding to a yield of 75.0%.

Elementary analysis:

found : 68.79% C, 10.08% H and 10.15% N

calcd. as $C_{16}H_{28}O_2N_2$ :

68.53% C; 10.07% H and 9.99% N.

IR :

$\nu_{max}^{nujol}$ 3000 - 2500 $cm^{-1}$ ($\nu$ $CO_2H$) and 1680 $cm^{-1}$ ($\nu$ C = O).

Mass : m/e 280 ($M^+$).

NMR (in $d_6$-DMSO) δ:

0.87 (3H, t, J = 6 Hz, $\underline{CH_3}(CH_2)_{10}$)

1.2 - 1.5 (16H, br. s, $CH_3\underline{(CH_2)_8}$ $(CH_2)_2$)

1.6 (2H, m, $C_9H_{19}$-$\underline{CH_2}$-$CH_2$)

2.62 (2H, t, J = 8 Hz, $C_{10}H_{21}$ - $\underline{CH_2}$)

2.80 (3H, s, N-$\underline{CH_3}$)

6.47 (1H, s, H in the pyrazole ring)

11.8 (1H, br. $CO_2H$ which disappears by treatment with $D_2O$)

EXAMPLE 21

Synthesis of 1-methyl-5-n-tridecylpyrazole-3-carboxylic acid:

In the same manner as in Example 18, 20.0 g of sodium 1-ethoxycarbonyl-oxo-1-hexadecenolate was cyclized to the corresponding N-methylpyrazole derivative. The crude product was recrystallized from a mixture of acetone and isopropyl ether to provide 1-methyl-5-n-tridecylpyrazole-3-carboxylic acid as colourless needle-like crystals melting at 82 to 83°C in an amount of 10.1 g corresponding to a yield of 57.2%.

Elementary analysis:

found : 70.26% C; 10.38% H and 8.88% N

calcd. as $C_{18}H_{32}O_2N_2$ :

70.09% C; 10.46% H and 9.08% N.

IR :

$\nu_{max}^{nujol}$ 3000 - 2500 $cm^{-1}$ ($\nu$ $CO_2H$) and 1680 $cm^{-1}$ ($\nu$ C = O)

Mass : m/e 308 ($M^+$).

NMR (in $d_6$-DMSO) δ:

0.87 (3H, t, J = 6 Hz, $CH_3(CH_2)_{12}$)

1.2 - 1.5 (20H, br. s, $CH_3(CH_2)_{10}(CH_2)_2$)

1.6 (2H, m, $C_{11}H_{23}-CH_2-CH_2$)

2.61 (2H, t, J = 8 Hz, $C_{12}H_{25}-CH_2$)

3.80 (3H, s, $N-CH_3$)

6.47 (1H, s, H in the pyrazole ring)

10.8 (1H, br., $CO_2H$ which disappears by treatment with $D_2O$)

EXAMPLE 22

Synthesis of 1-phenyl-5-n-nonylpyrazole-3-carboxylic acid:

10.0 g (0.034 mol) of sodium 1-ethoxycarbonyl-3-oxo-1-dodecenolate and 8.2 g (0.026 mol) of phenylhydrazine sulfate were dissolved in 40 ml of methanol, and 20 ml of saturated methanolic solution of sodium hydroxide were added dropwise. After the addition, the mixture was gently heated for 2 hours under reflux and after making it acidic with concentrated hydrochloric acid and adding distilled water, methanol was removed under a reduced pressure. The residue was extracted with chloroform, and the chloroform layer was washed with a saturated aqueous solution of sodium chloride and dried over anhydrous sodium sulfate and concentrated. The residue was recrystallized from ethanol to provide 5.0 g of the desired product as colourless needle-like crystals melting at 87 to 88°C corresponding to a yield of 61.7%.

Elementary analysis:

found : 72.47% C; 8.05% H and 8.84% N

calcd. as $C_{19}H_{26}O_2N_2$ :

72.58% C; 8.34% H and 8.91% N.

IR :

$\nu_{max}^{nujol}$ 3000 - 2500 $cm^{-1}$ ($\nu$ $CO_2H$), 1700 $cm^{-1}$ ($\nu$ C = O) and 1600 $cm^{-1}$ ($\nu$ benzene ring)

Mass : m/e 314 ($M^+$).

NMR (in $d_6$-DMSO) δ:

0.85 (3H, t, J = 6 Hz, $CH_3(CH_2)_8$)

1.2 - 1.5 (12H, br. s, $CH_3(CH_2)_6(CH_2)_2$)

1.6 (2H, m, $C_7H_{15}$-$CH_2$-$CH_2$)

2.52 (2H, t, J = 8 Hz, $C_2H_{17}$- $CH_2$)

6.67 (1H, s, H in the pyrazole ring)

7.51 (5H, m, 5 X H in the benzene ring)

11.0 (1H, br., $CO_2H$, disappearing by treatment with $D_2O$)

EXAMPLE 23

Synthesis of 1-phenyl-5-n-heptylpyrazole-3-carboxylic acid:

In the same manner as in Example 18, 9.0 g (0.034 mol) of sodium 1-ethoxycarbonyl-3-oxo-1-decenolate was reacted with 8.2 g (0.026 mol) of phenylhydrazine sulfate to give the corresponding 1-phenylpyrazole derivative as a crude oil. After treatment with activated carbon, the residue was distilled under a reduced pressure to provide 5.5 g of a colourless oil with a boiling point of 97 to 98°C/0.28 mmHg at a yield of 56.7%.

Elementary analysis:

found: 71.40% C; 7.47% H and 9.95% N

calcd. as $C_{17}H_{22}O_2N_2$:
71.30% C; 7.74% H and 9.78% N.

IR :

$\nu_{max}^{nujol}$ 3000 - 2500 $cm^{-1}$ ($\nu$ $CO_2H$), 1700 $cm^{-1}$ ($\nu$ C = O) and 1600 $cm^{-1}$ ($\nu$ benzene ring).

Mass: m/e 286 ($M^+$).

NMR (in $CDCl_3$) δ:

0.89 (3H, t, J = 6 Hz, $\underline{CH_3}(CH_2)_6$)

1.2 - 1.5 (8H, br. $CH_3\underline{(CH_2)_4}-(CH_2)_2$)

1.6 (2H, m, $C_5H_{11}-\underline{CH_2}-CH_2$)

2.60 (2H, t, J = 8 Hz, $C_6H_{13}-$ $\underline{CH_2}$)

6.72 (1H, s, H in the pyrazole ring)

7.40 (5H, br. s, 5 X H in the benzene ring)

10.8 (1H, br., $CO_2H$ disappearing by treatment with $D_2O$).

EXAMPLE 24

Synthesis of 1-phenyl-5-n-undecylpyrazole-3-carboxylic acid:

In the same manner as in Example 18, 11.0 g (0.034 mol) of sodium 1-ethoxycarbonyl-3-oxo-1-tetradecenolate was reacted with 8.2 g (0.026 mol) of phenylhydrazine sulfate to give the corresponding 1-phenylpyrazole derivative. The crude product was recrystallized from ethanol to provide 8.1 g of colourless needle-like crystals melting at 53 to 55°C corresponding to a yield of 69.7%.

Elementary analysis:

found: 73.70% C; 8.91% H and 8.40% N

calcd. as $C_{21}H_{30}O_2N_2$:

73.64% C; 8.83% H and 8.18% N.

IR:

$\nu_{max}^{nujol}$ 3000 - 2500 $cm^{-1}$ ($\nu$ $CO_2H$), 1700 $cm^{-1}$ ($\nu C = O$) and 1600 $cm^{-1}$ ($\nu$ benzene ring).

Mass: m/e 342 ($M^+$).

NMR (in $d_6$-DMSO) $\delta$:

0.86 (3H, t, J = 6 Hz, $\underline{CH_3}(CH_2)_{10}$)

1.2 - 1.5 (16H, br. s, $CH_3\underline{(CH_2)_8}(CH_2)_2$)

1.6 (2H, m, $C_9H_{19}$-$\underline{CH_2}$-$CH_2$)

2.65 (2H, t, J = 8 Hz, $C_{10}H_{21}$ - $\underline{CH_2}$)

6.70 (1H, s, H in the pyrazole ring)

7.52 (5H, br. s, 5 X H in the benzene ring)

12.3 (1H, br., $CO_2H$ disappearing by treatment with $D_2O$)

EXAMPLE 25

Synthesis of 1-phenyl-5-n-tridecylpyrazole -3-carboxylic acid:

In the same manner as in Example 18, 9.0 g (0.026 mol) of sodium 1-ethoxycarbonyl-3-oxo-1-hexadecenolate was reacted with 6.3 g (0.020 mol) of phenylhydrazine sulfate to give the corresponding 1-phenylpyrazole derivative. The crude product was recrystallized from ethanol to provide colourless needle-like crystals melting at 89 to 90°C in an amount of 4.4 g corresponding to a yield of 59.8%.

Elementary analysis:

found: 74.08% C; 9.52% H and 7.59% N

calcd. as $C_{23}H_{34}O_2N_2$ :

74.55% C; 9.25% H and 7.56% N.

IR :

$\nu_{max}^{nujol}$ 3000 - 2500 $cm^{-1}$ ($\nu$ $CO_2H$), 1700 $cm^{-1}$ ($\nu$ C = O) and 1600 $cm^{-1}$ ($\nu$ benzene ring)

Mass: m/e 370 ($M^+$).

NMR (in $CDCl_3$) $\delta$:

0.87 (3H, t, J = 6 Hz, $\underline{CH_3}(CH_2)_{12}$)

1.2 - 1.5 (20H, br. s, $CH_3\underline{(CH_2)_{10}}(CH_2)_2$)

1.7 (2H, m, $C_{11}H_{23}$-$\underline{CH_2}CH_2$)

2.60 (2H, t, J = 8 Hz, $C_{12}H_{25}$ - $\underline{CH_2}$)

6.58 (1H, s, H in the pyrazole ring)

7.4 - 7.8 (5H, m, 5 X H in the benzene ring)

12.0 (1H, br., $CO_2H$ disappearing by treatment with $D_2O$)

Figures 1 to 25 of the accompanying drawings show the infrared absorption spectra of the pyrazole derivatives prepared in accordance with Examples 1 to 25, the number of each Figure corresponding to the number of the appropriate Example.

EXAMPLE 26

200 G of 5-n-tridecylpyrazole-3-carboxylic acid, 50 g of lactose, 20 g of potato starch, 15 g of CMC and 10 g of methylcellulose were mixed uniformly, the mixture was shaped into particles by introducing the mixture together with an aqueous 50% ethanolic solution into a tumbling forming machine, and the thus obtained particles were dried using a fluidizing dryer. After drying, the particles were sifted by screening machine provided with a sieve having a sieve opening of 500 microns (32 mesh Tyler standard). The particles passing through the sieve were mixed with 3 g of talc and 2 g of magnesium stearate and the mixture was pressed in a rotary tabletting machine into tablets each 9 mm in diameter and 300 mg in weight.

EXAMPLE 27

Particles were prepared by the same procedure as in Example 26, but using ethyl 5-n-tridecylpyrazole-3-carboxylate instead of the acid itself used in Example 26, and the particles were capsulated in No. 1 hard capsules by using a capsulating machine at a rate of 300 mg/capsule.

The pyrazole derivatives of general formula (I) can in general be prepared by reacting a sodium enolate of the formula

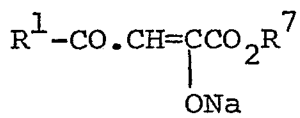

wherein $R^7$ represents a lower alkyl group with hydrazine hydrate or a hydrazine sulfate of the formula $R^2NH.NH_2.H_2SO_4$ to form a 5-alkylpyrazole-3-carboxylic acid or an alkyl ester thereof of the formula

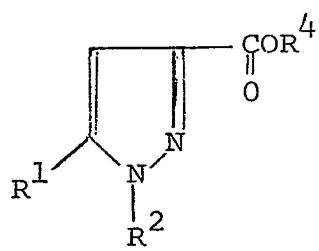

wherein $R^4$ is hydrogen or a lower alkyl group, and if desired, when $R^4$ is lower alkyl converting the alkyl ester to the corresponding acid wherein $R^4$ is hydrogen by hydrolysis or to a pyrazole derivative in which $R^3$ is $-CH_2OH$ by reduction, or when $R^4$ is hydrogen converting the carboxylic acid to a pyrazole derivative in which $R^3$ is 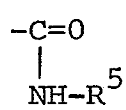 by forming the acid chloride from the carboxylic acid and reacting the acid chloride with an amine derivative of the formula $NH_2R^5$ or a hydrochloride thereof or to the alkyl ester wherein $R^4$ is lower alkyl by esterification.

CLAIMS

1. A pyrazole derivative of the general formula:

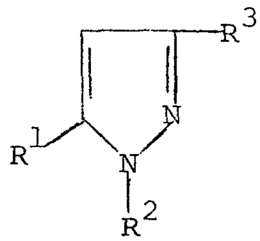

wherein $R^1$ represents an alkyl group of 7 to 17 carbon atoms, $R^2$ represents a hydrogen atom, a lower alkyl group or a phenyl group and $R^3$ represents

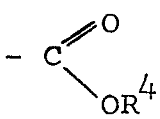 , 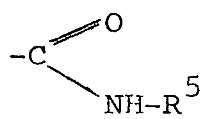 or a hydroxymethyl group,

wherein $R^4$ represents a hydrogen atom or a lower alkyl group and $R^5$ represents $-CH_2CH_2OH$ or 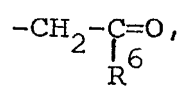 ,

wherein $R^6$ represents a hydroxyl group or a lower alkoxy group, with the proviso that when $R^1$ represents an alkyl group of 7 to 11 carbon atoms and $R^2$ represents a hydrogen atom, $R^3$ does not represent

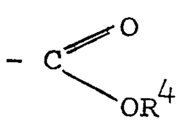 .

2. A pyrazole derivative according to claim 1 which is a 5-alkylpyrazole-3-carboxylic acid of the formula:

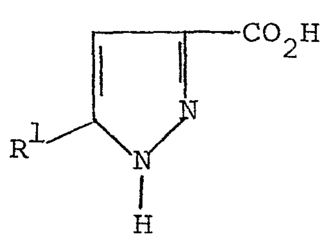

wherein $R^1$ represents an alkyl group of 13 to 17 carbon atoms.

3. A pyrazole derivative according to claim 1 which is a 5-alkylpyrazole-3-carboxylic acid ester of the formula:

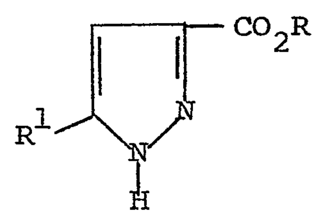

wherein R represents a lower alkyl group and $R^1$ represents an alkyl group of 13 to 17 carbon atoms.

4. A pyrazole derivative according to claim 1 which is a 3-N-2'-hydroxyethylcarbamoyl-pyrazole of the formula:

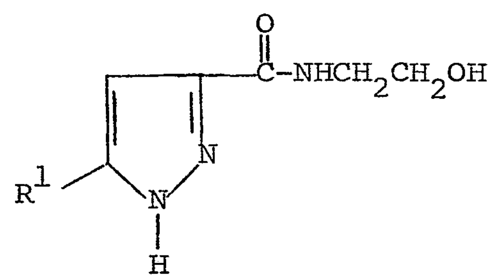

wherein $R^1$ represents an alkyl group of 9 to 17 carbon atoms.

5. A pyrazole derivative according to claim 1 which is a 3-N-alkoxycarbonylmethylcarbamoyl-pyrazole of the formula:

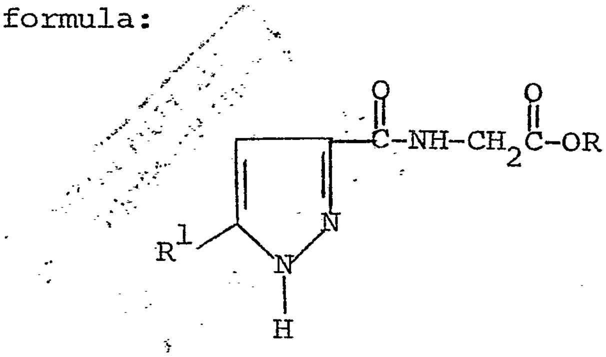

wherein R represents a lower alkyl group and $R^1$ represents an alkyl group of 9 to 13 carbon atoms.

6. A pyrazole derivative according to claim 1 which is a 3-hydroxymethylpyrazole of the formula:

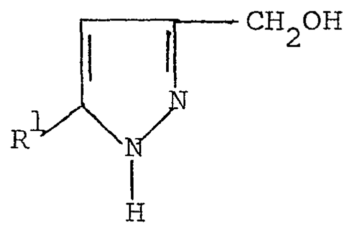

wherein $R^1$ represents an alkyl group of 7 to 13 carbon atoms.

7. A pyrazole derivative according to claim 1 which is a 1-methyl-5-alkylpyrazole-3-carboxylic acid of the formula:

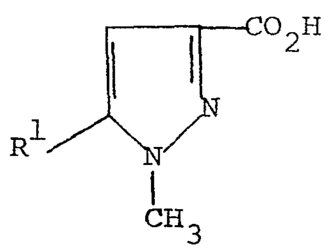

wherein $R^1$ represents an alkyl group of 7 to 13 carbon atoms.

8. A pyrazole derivative according to claim 1 which is a 1-phenyl-5-alkylpyrazole-3-carboxylic acid of the formula:

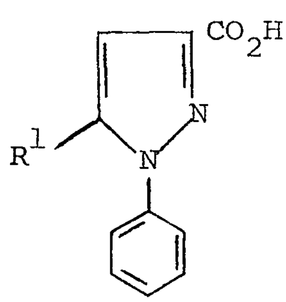

wherein $R^1$ represents an alkyl group of 7 to 13 carbon atoms.

9. A pharmaceutical composition comprising, as active ingredient, a pyrazole derivative as claimed in any one of claims 1 to 8, together with a pharmaceutically acceptable carrier.

CLAIMS FOR AUSTRIA

1. A process for preparing pyrazole derivative of the general formula:

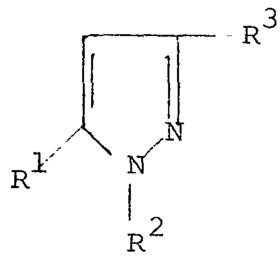

wherein $R^1$ represents an alkyl group of 7 to 17 carbon atoms, $R^2$ represents a hydrogen atom, a lower alkyl group or a pehnyl group and $R^3$ represents

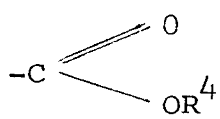 , 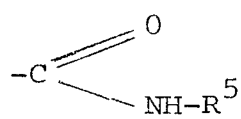 or a hydroxymethyl group, wherein $R^4$ represents a hydrogen atom or a lower alkyl group and $R^5$ represents $-CH_2CH_2OH$ or $-CH_2-\underset{R^6}{C}=0$, wherein $R^6$ represents a hydroxyl group or a lower alkoxy group, with the proviso that when $R^1$ represents an alkyl group of 7 to 11 carbon atoms and $R^2$ represents a hydrogen atom, $R^3$ does not represent

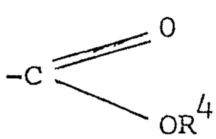 , the process being characterised by reacting a sodium enolate of the formula

$$R^1-CO.CH=\underset{ONa}{C}CO_2R^7$$

wherein $R^7$ represents a lower alkyl group with hydrazine hydrate or a hydrazine sulfate of the formula $R^2NH.NH_2.H_2SO_4$ to form a 5-alkylpyrazole-3-carboxylic acid or an alkyl ester thereof of the formula

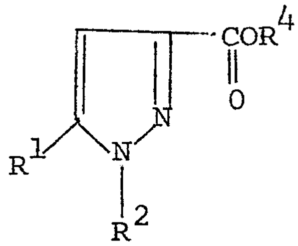

wherein $R^4$ is hydrogen or a lower alkyl group, and, if desired, when $R^4$ is lower alkyl converting the alkyl ester to the corresponding acid wherein $R^4$ is hydrogen by hydrolysis or to a pyrazole derivative in which $R^3$ is $-CH_2OH$ by reduction, or when $R^4$ is hydrogen converting the carboxylic acid to a pyrazole derivative in which $R^3$ is $-C(=O)-NH-R^5$ by forming the acid chloride from the carboxylic acid and reacting the acid chloride with an amine derivative of the formula $NH_2R^5$ or a hydrochloride thereof or to the alkyl ester wherein $R^4$ is lower alkyl by esterification.

2. A process according to claim 1 characterised in that an alkyl ester of a 5-alkylpyrazole-3-carboxylic acid is prepared by reacting the sodium enolate with hydrazine hydrate in a mineral acid, and if desired the carboxylic acid is prepared by hydrolysing the alkyl ester.

3. A process according to claim 1 characterised in that a 5-alkylpyrazole-3-carboxylic acid is prepared by reacting the sodium enolate with hydrazine sulfate in an aqueous solution of sodium hydroxide.

4. A process according to claim 3 characterised in that a 5-alkylpyrazol-3-carboxamide is formed by reacting the 5-alkylpyrazole-3-carboxylic acid with thionyl chloride to provide the carboxylic acid chloride and reacting the carboxylic acid chloride with the amine derivative of formula $NH_2R^5$ or hydrochloride thereof in dichloroethane in the presence of triethylamine.

5. A process according to claim 1 characterised in that a 3-hydroxymethyl-5-alkylpyrazole is prepared by reducing the alkyl ester of a 5-alkylpyrazole-3-carboxylic acid with lithium aluminium hydride in anhydrous tetrahydrofuran.

6. A process according to claim 1 characterised in that a 1-alkyl or phenyl-5-alkylpyrazole-3-carboxylic acid is produced by dissolving the sodium enolate in water or methanol together with a hydrazine sulfate of the formula

$$R^2NH.NH_2.H_2SO_4$$

wherein $R^2$ is lower alkyl or phenyl, adding sodium hydroxide to the resulting solution and heating the mixture.

0029363

FIG. 1

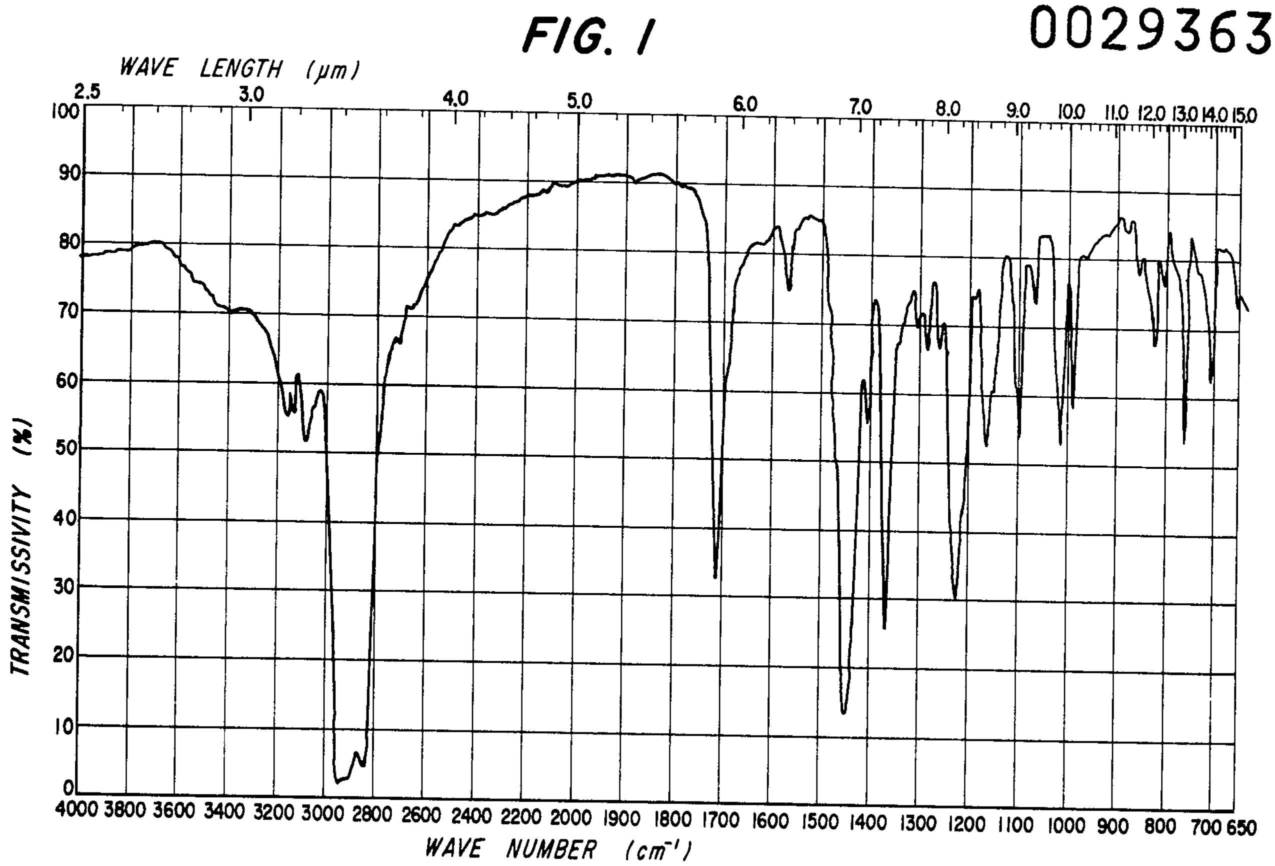


FIG. 2

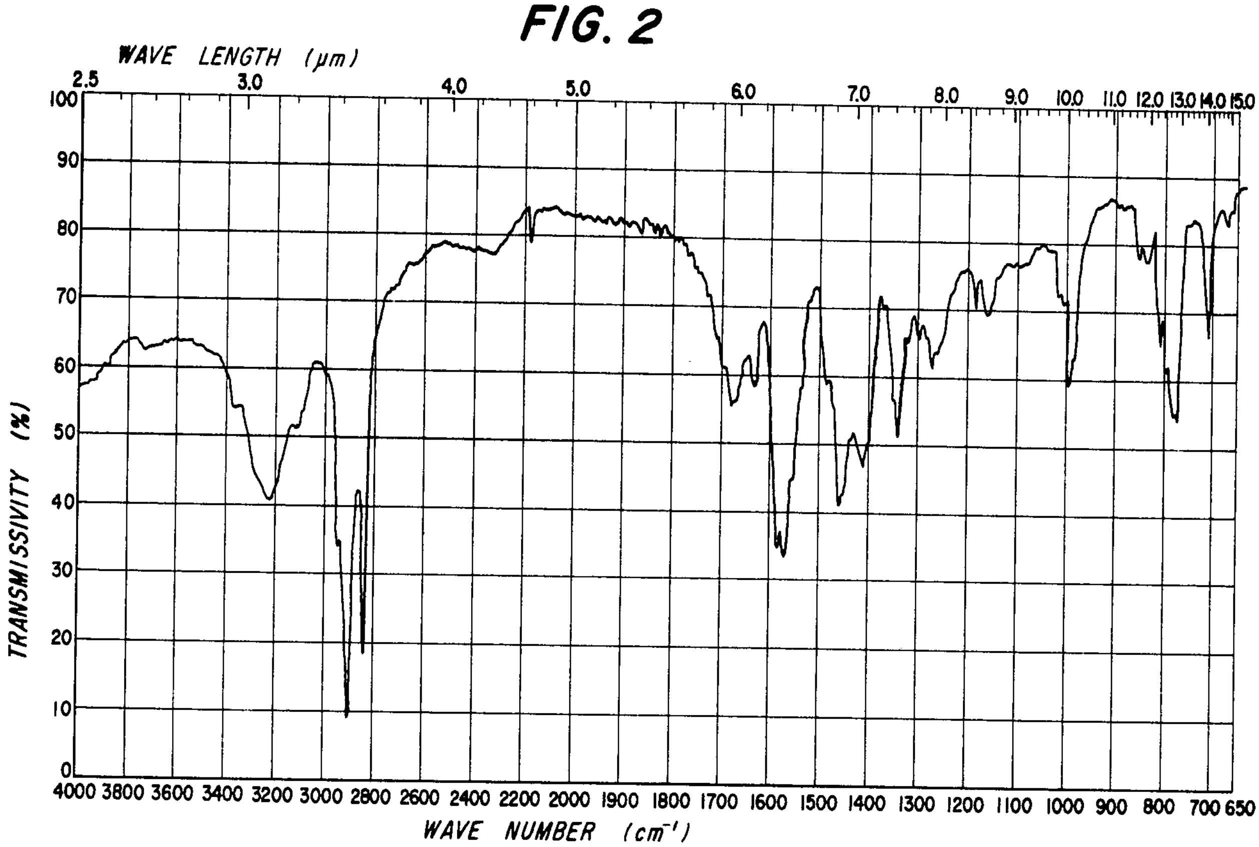

2/13

0029363

## FIG. 3

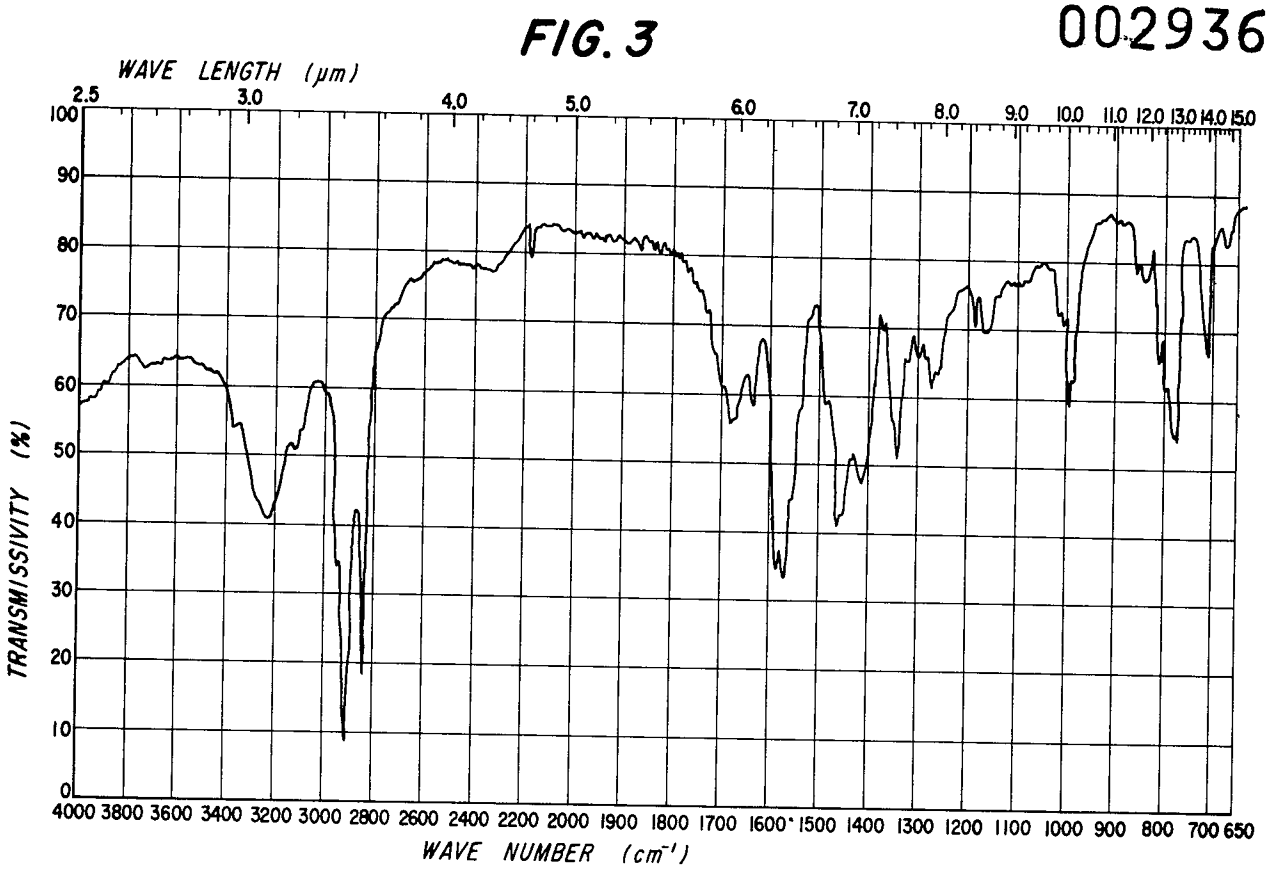


## FIG. 4

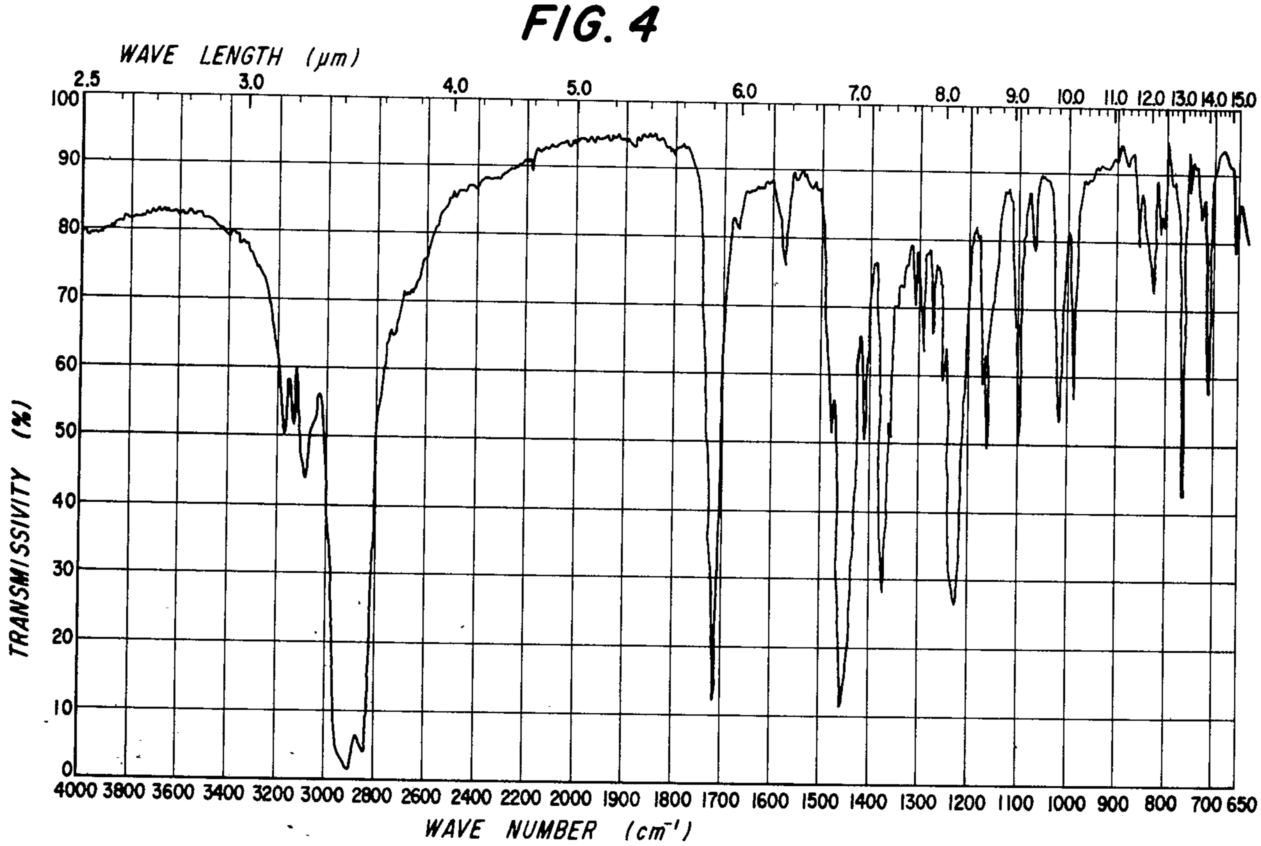

FIG. 5

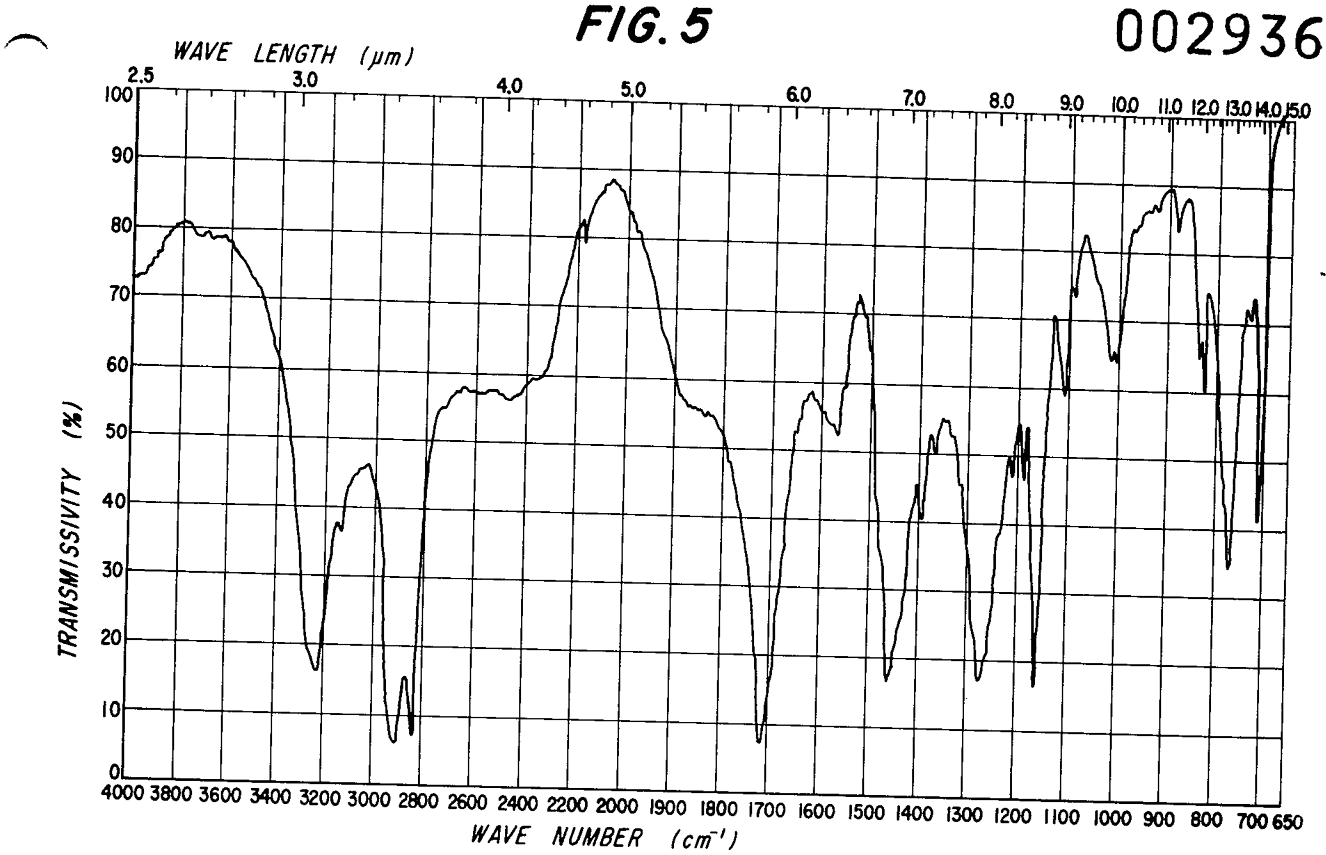
WAVE LENGTH (μm)
2.5 3.0 4.0 5.0 6.0 7.0 8.0 9.0 10.0 11.0 12.0 13.0 14.0 15.0
TRANSMISSIVITY (%)
0 10 20 30 40 50 60 70 80 90 100
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

FIG. 6

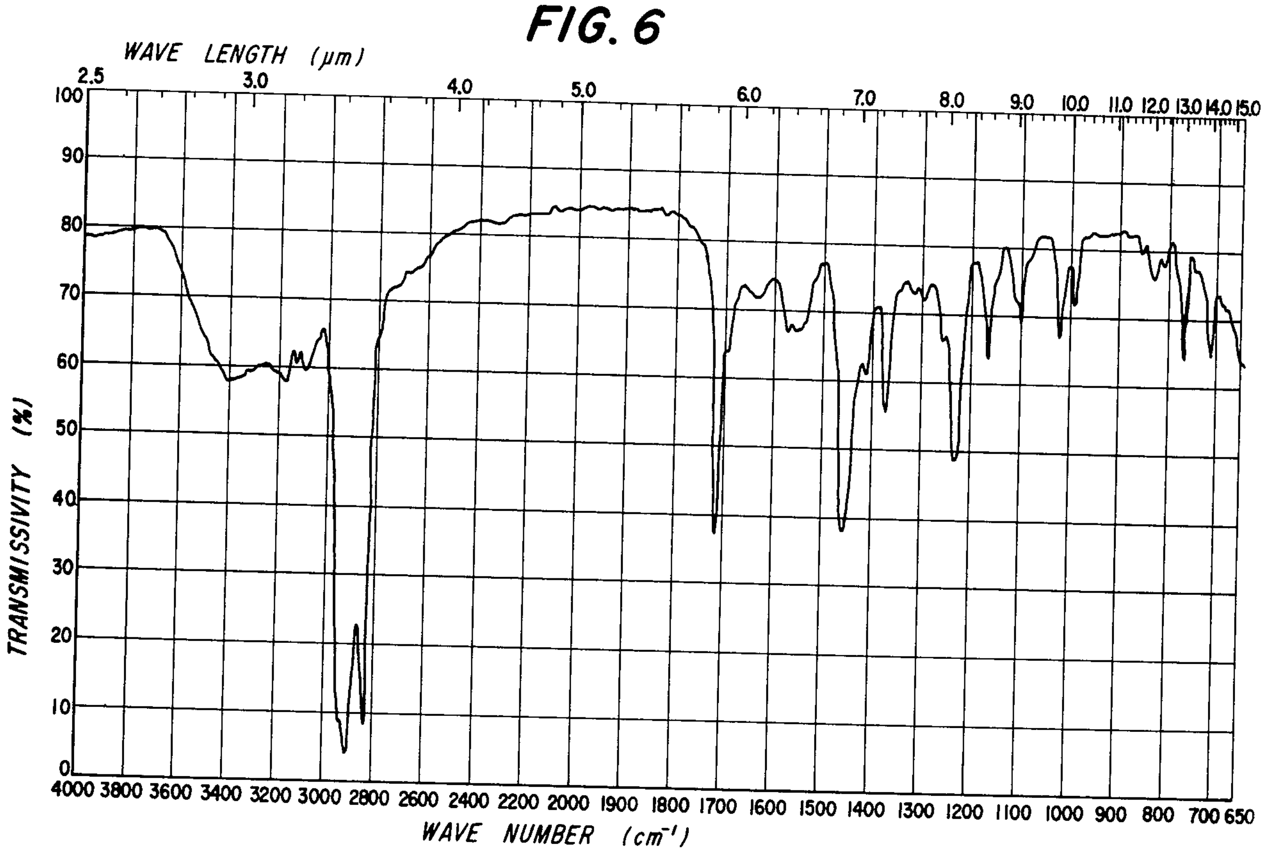
WAVE LENGTH (μm)
2.5 3.0 4.0 5.0 6.0 7.0 8.0 9.0 10.0 11.0 12.0 13.0 14.0 15.0
TRANSMISSIVITY (%)
0 10 20 30 40 50 60 70 80 90 100
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

M(C-2

4/13

0029363

FIG. 7

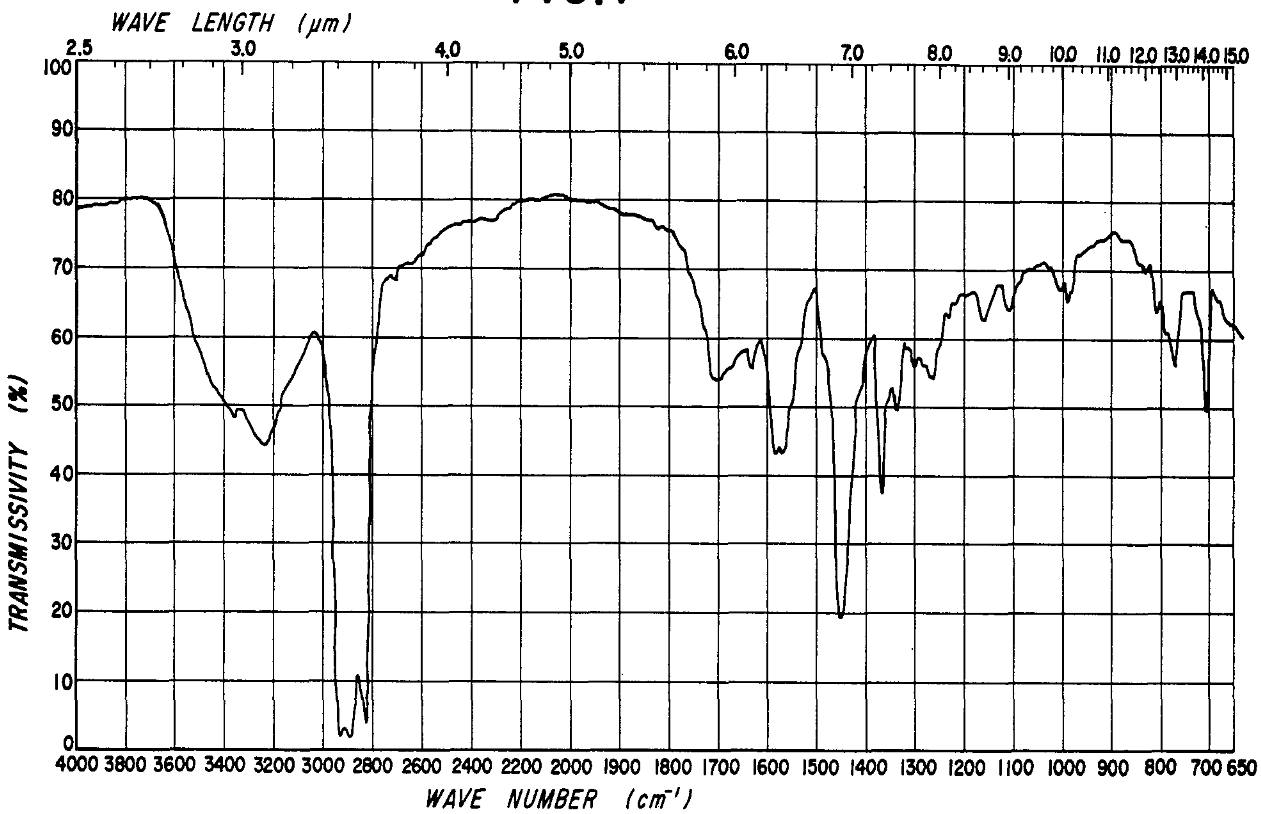


FIG. 8

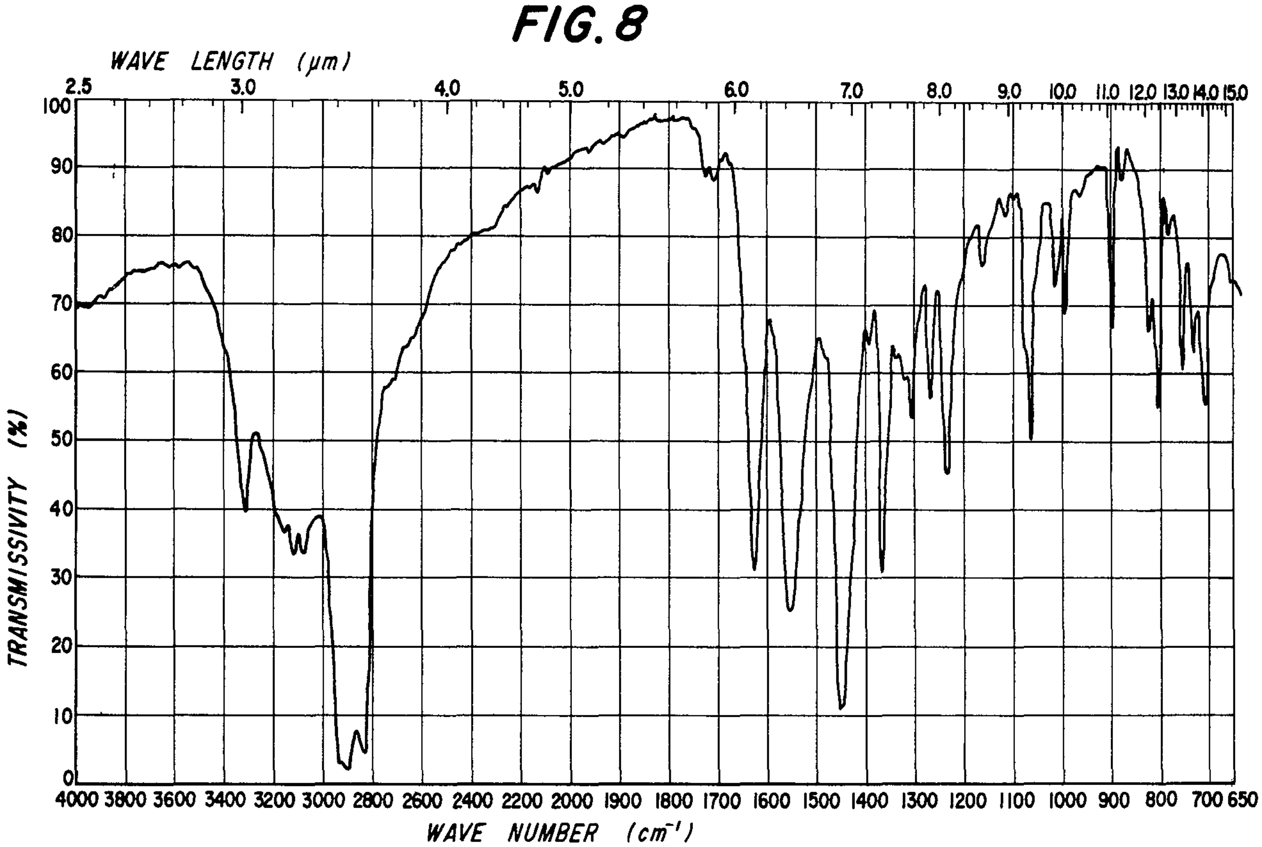

FIG. 9

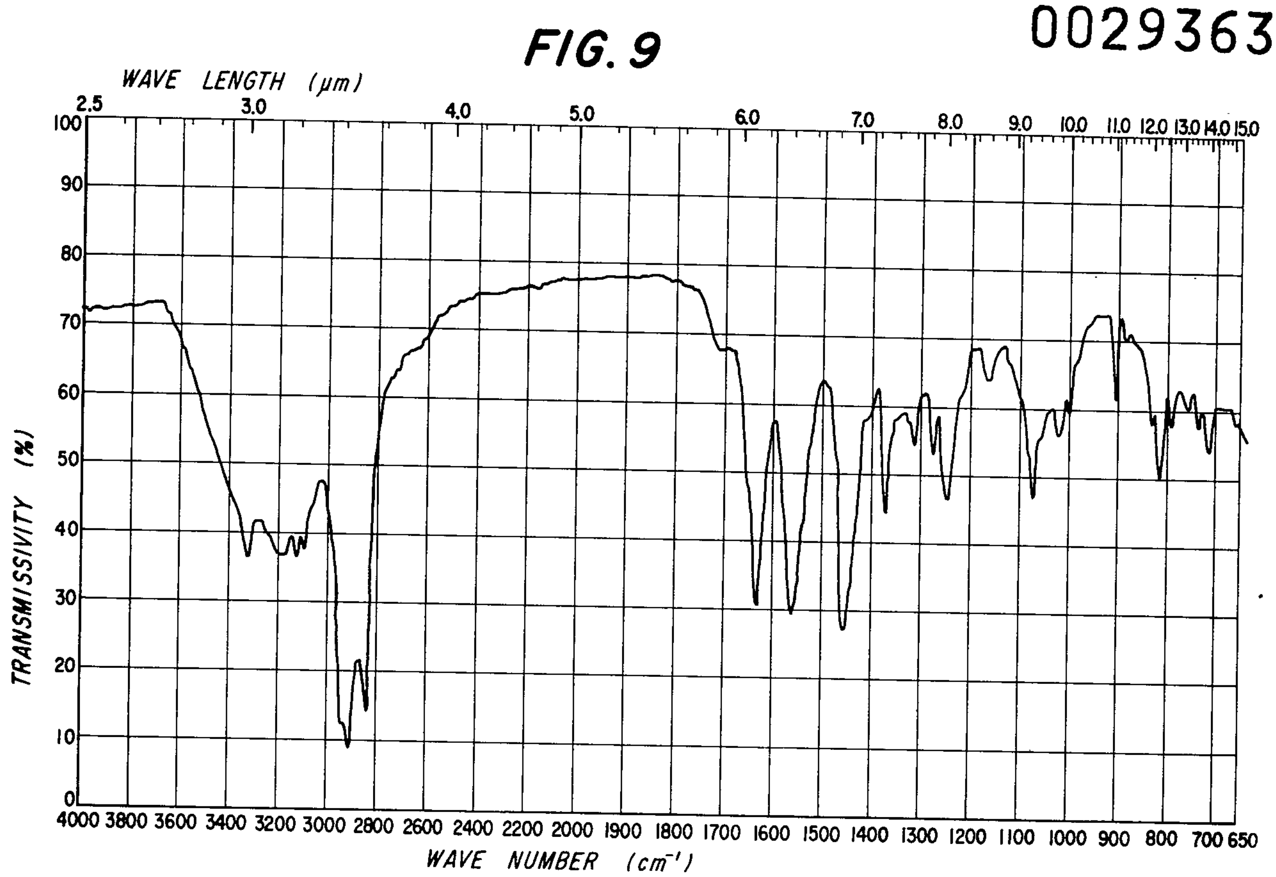
WAVE LENGTH (μm)
2.5
3.0
4.0
5.0
6.0
7.0
8.0
9.0
10.0
11.0
12.0
13.0
14.0
15.0
TRANSMISSIVITY (%)
100
90
80
70
60
50
40
30
20
10
0
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

FIG. 10

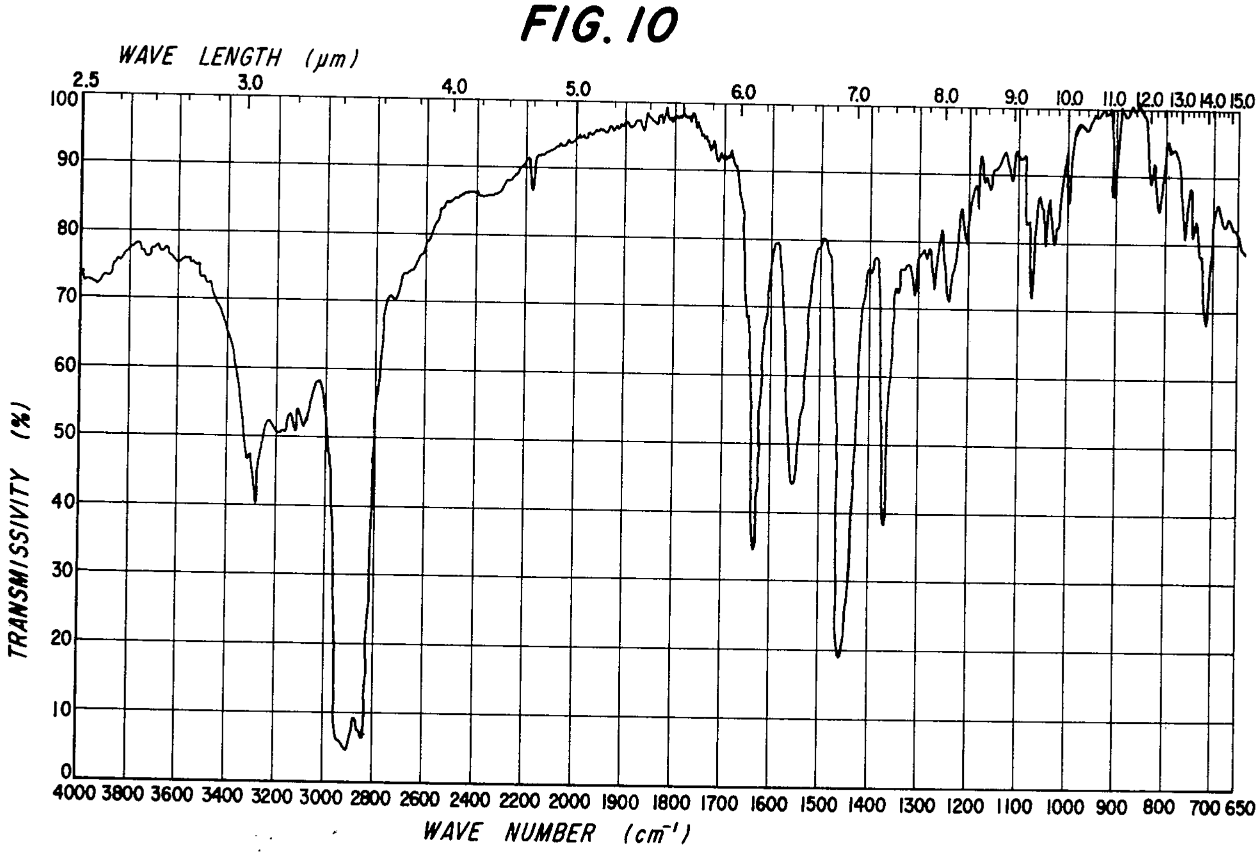
WAVE LENGTH (μm)
2.5
3.0
4.0
5.0
6.0
7.0
8.0
9.0
10.0
11.0
12.0
13.0
14.0
15.0
TRANSMISSIVITY (%)
100
90
80
70
60
50
40
30
20
10
0
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

MCC-

0029363

FIG. 11

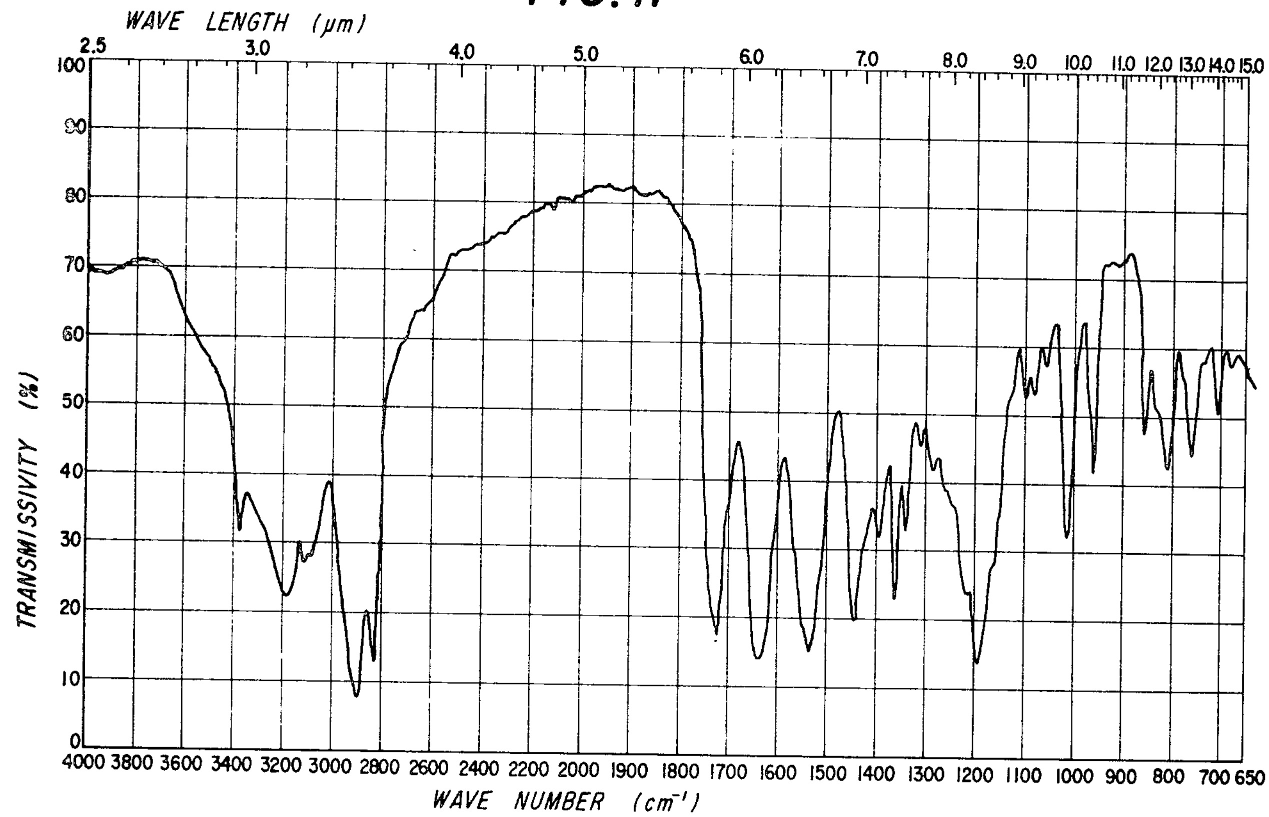


FIG. 12

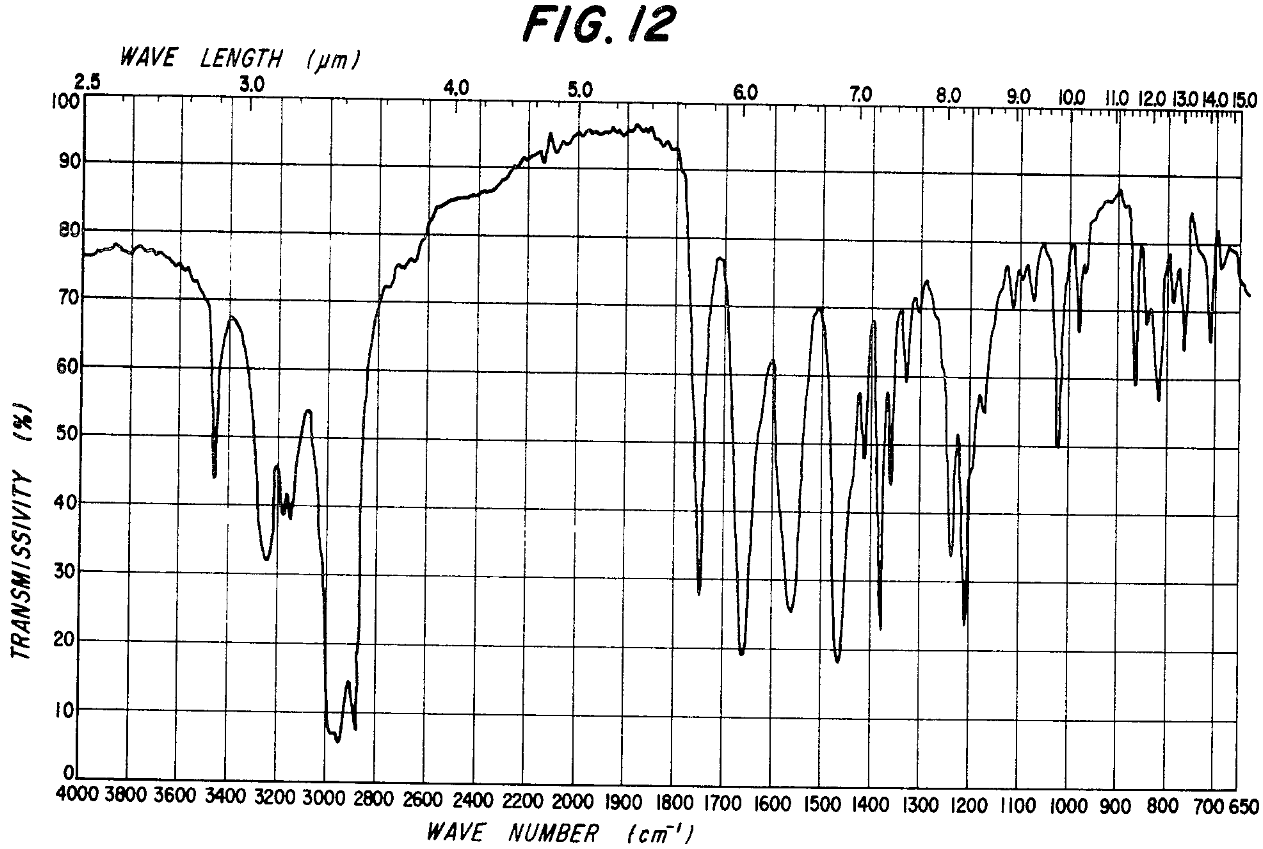

7/13

0029363

FIG. 13

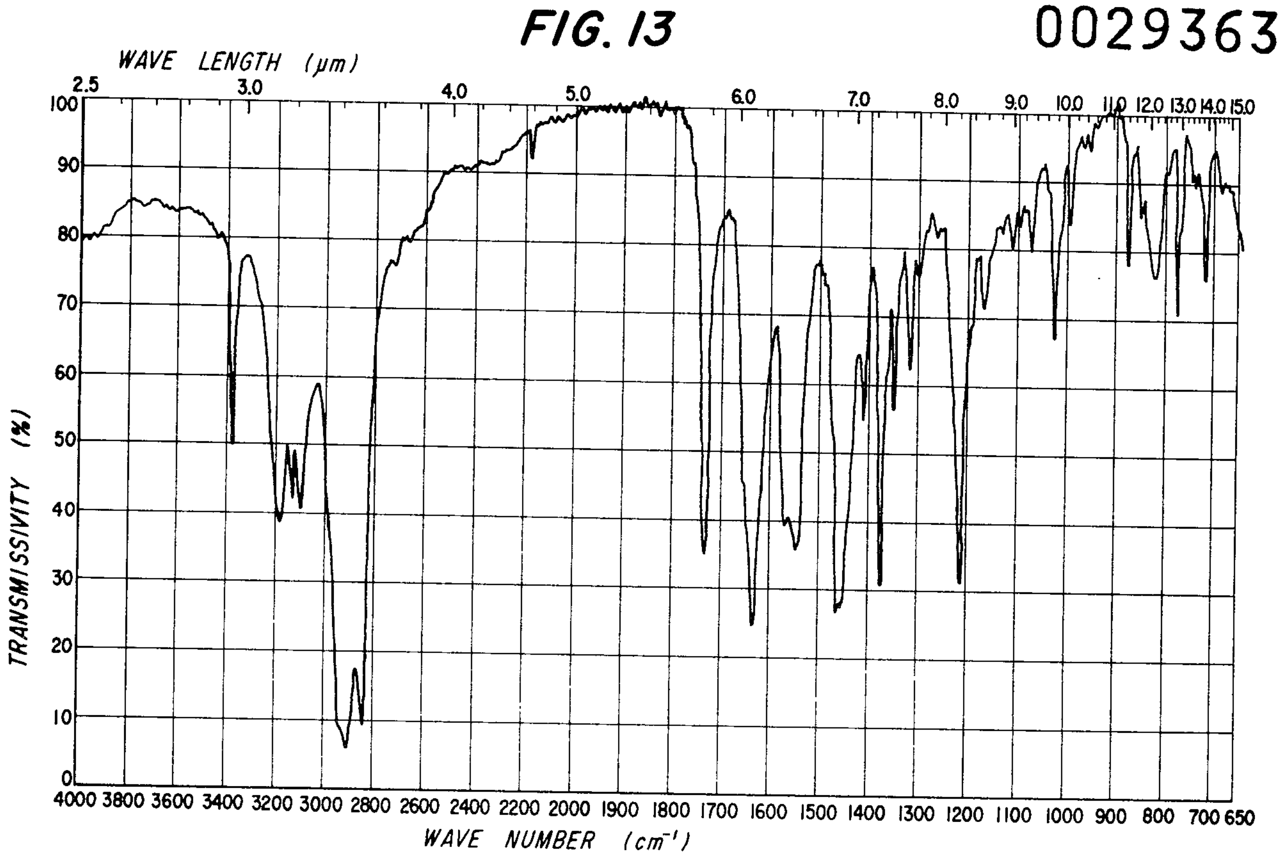


FIG. 14

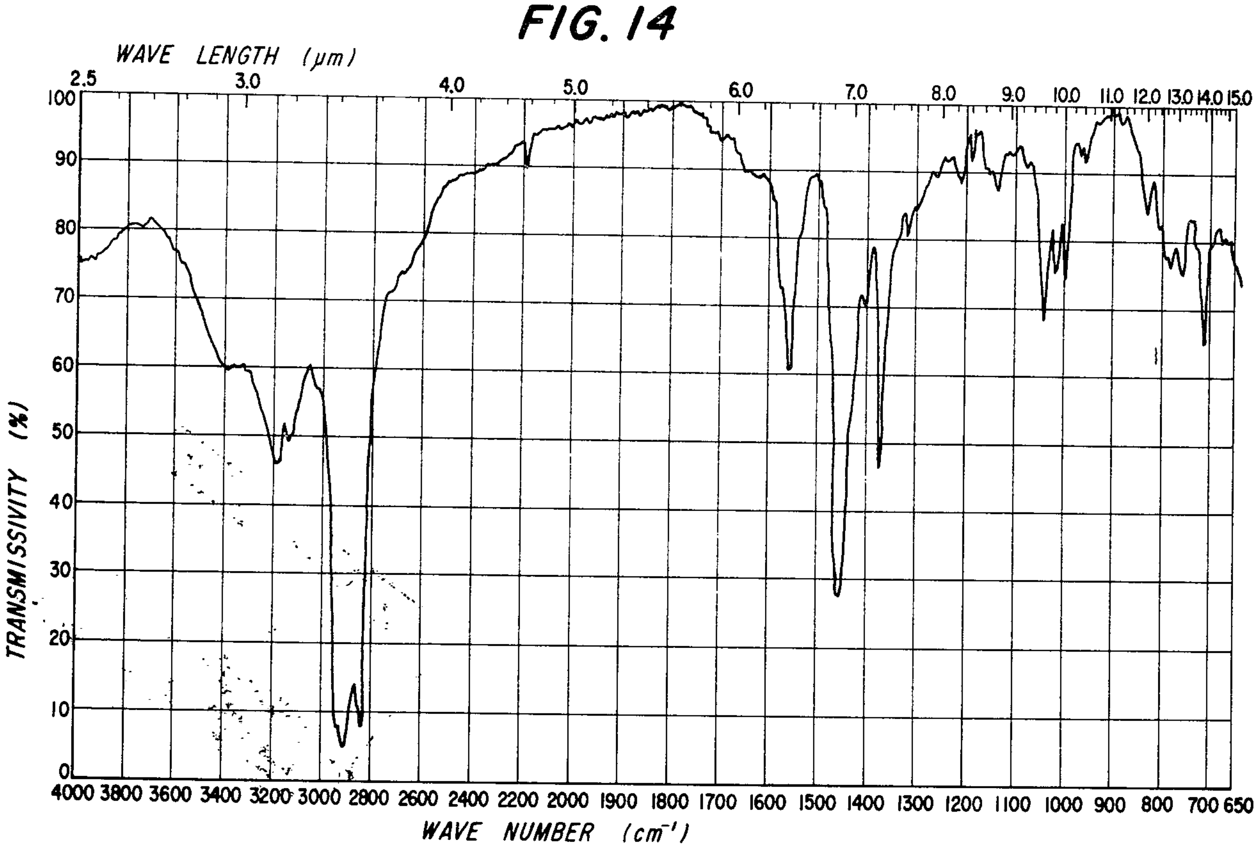

0029363

FIG. 15

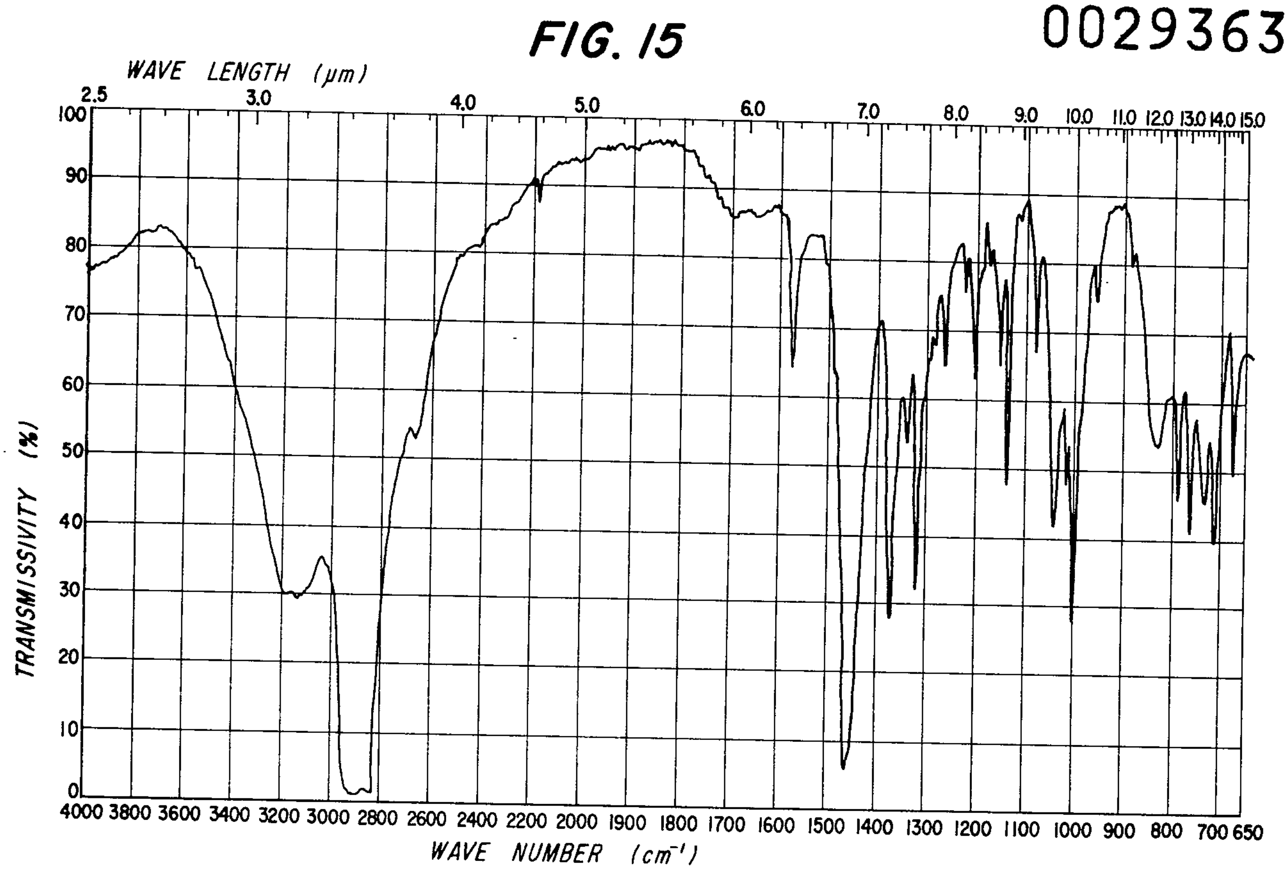


FIG. 16

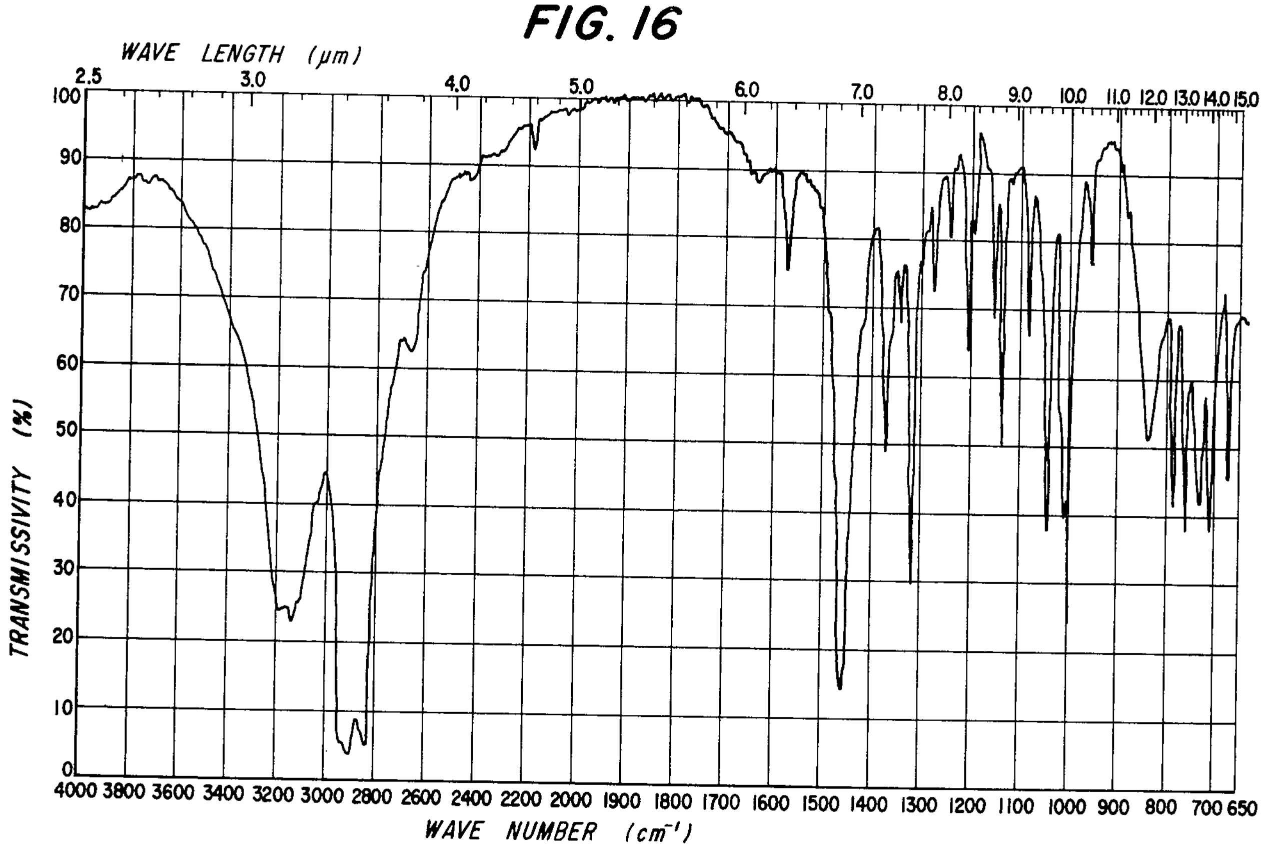

FIG. 17

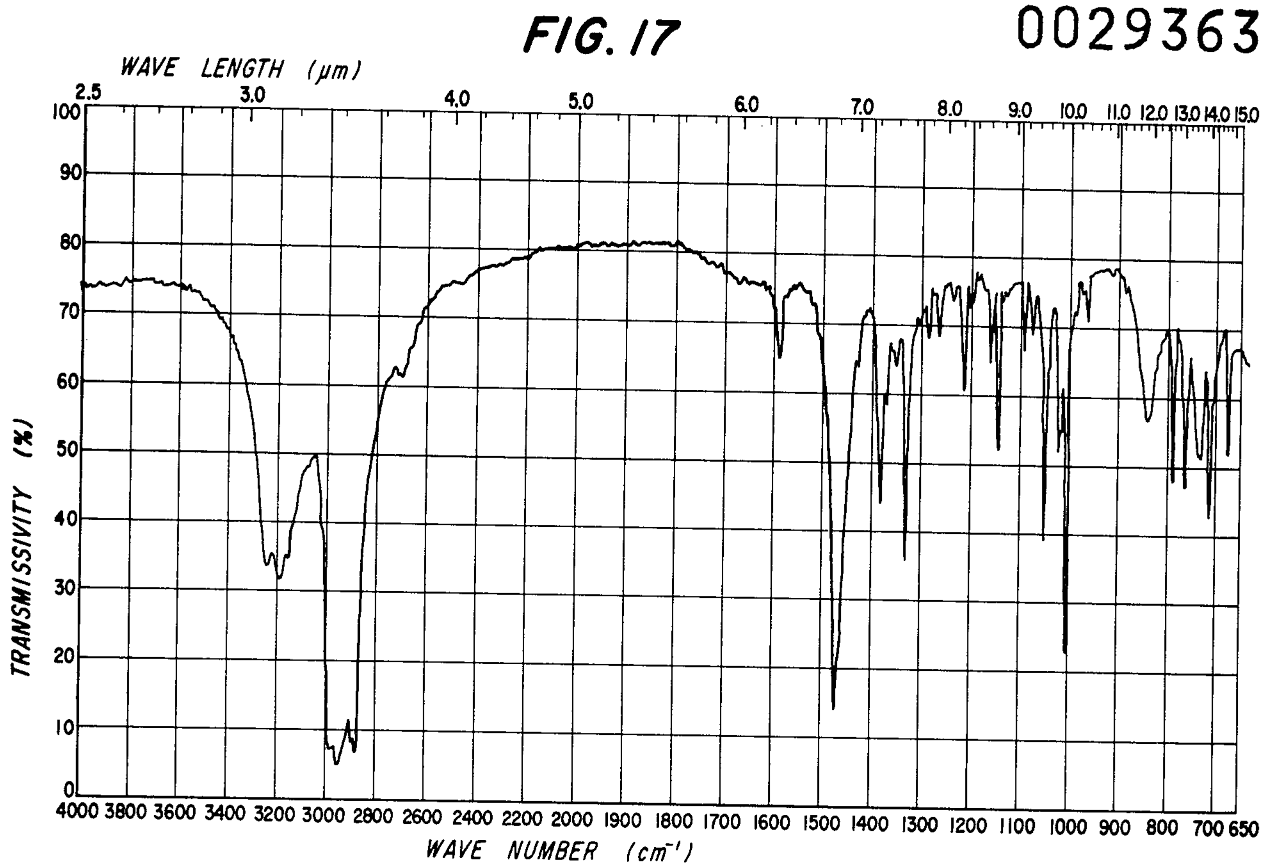
WAVE LENGTH (μm)
2.5 3.0 4.0 5.0 6.0 7.0 8.0 9.0 10.0 11.0 12.0 13.0 14.0 15.0
TRANSMISSIVITY (%)
100 90 80 70 60 50 40 30 20 10 0
4000 3800 3600 3400 3200 3000 2800 2600 2400 2200 2000 1900 1800 1700 1600 1500 1400 1300 1200 1100 1000 900 800 700 650
WAVE NUMBER ($cm^{-1}$)

FIG. 18

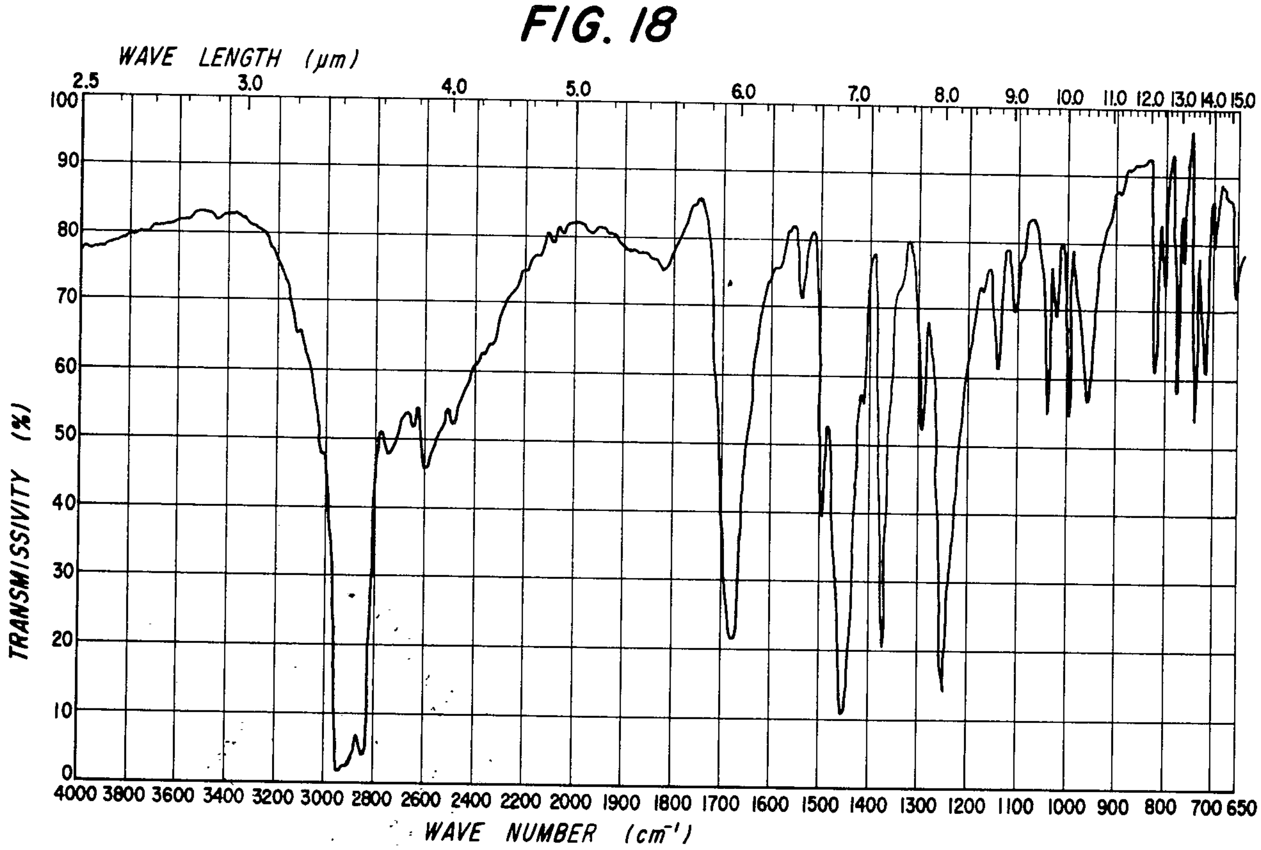
WAVE LENGTH (μm)
2.5 3.0 4.0 5.0 6.0 7.0 8.0 9.0 10.0 11.0 12.0 13.0 14.0 15.0
TRANSMISSIVITY (%)
100 90 80 70 60 50 40 30 20 10 0
4000 3800 3600 3400 3200 3000 2800 2600 2400 2200 2000 1900 1800 1700 1600 1500 1400 1300 1200 1100 1000 900 800 700 650
WAVE NUMBER ($cm^{-1}$)

0029363

FIG. 19

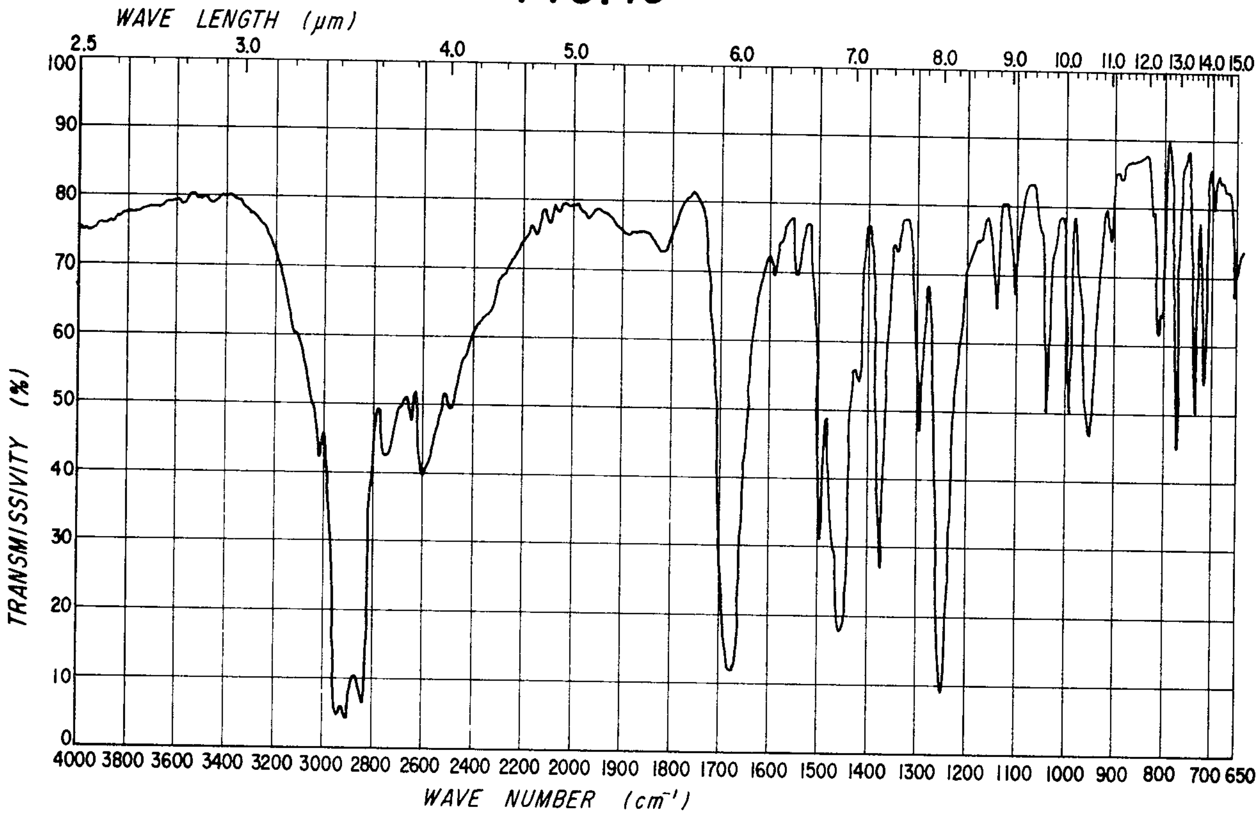


FIG. 20

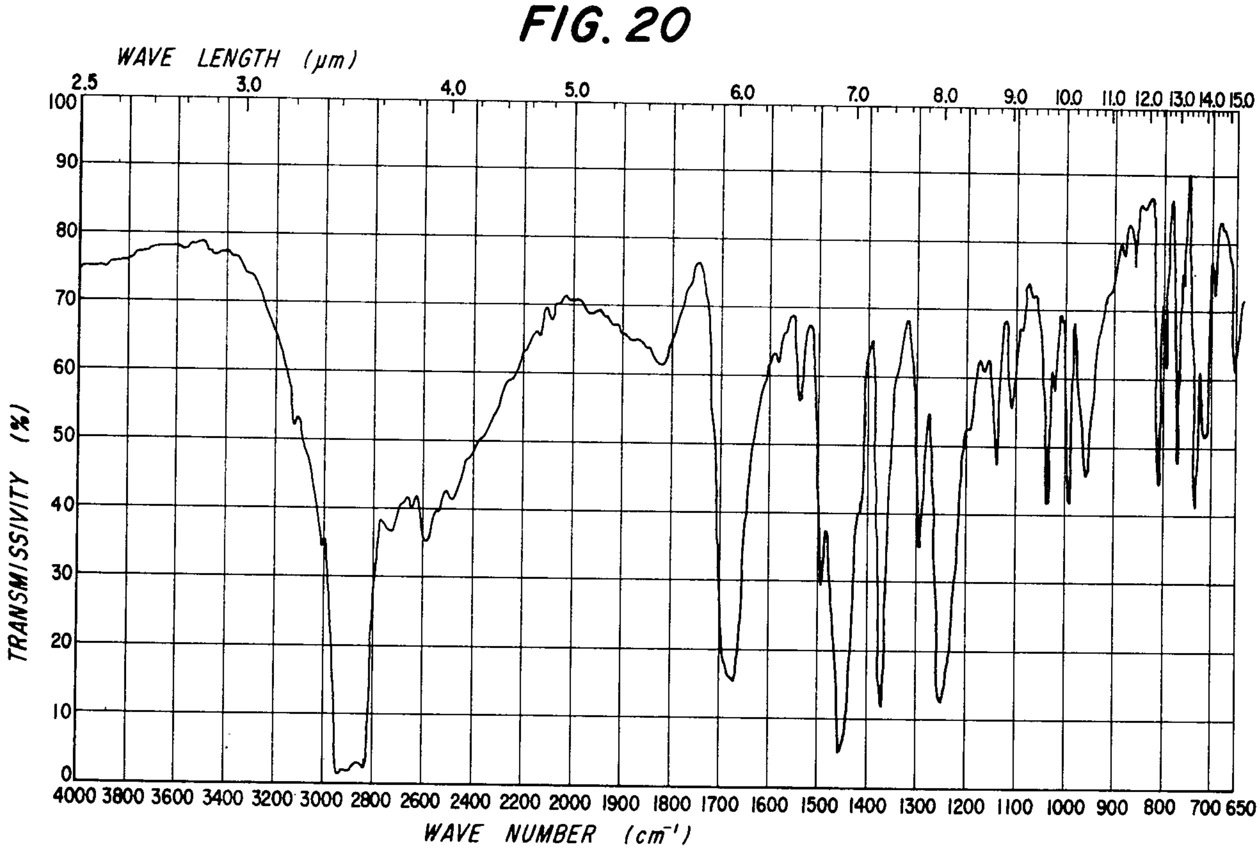

FIG. 21

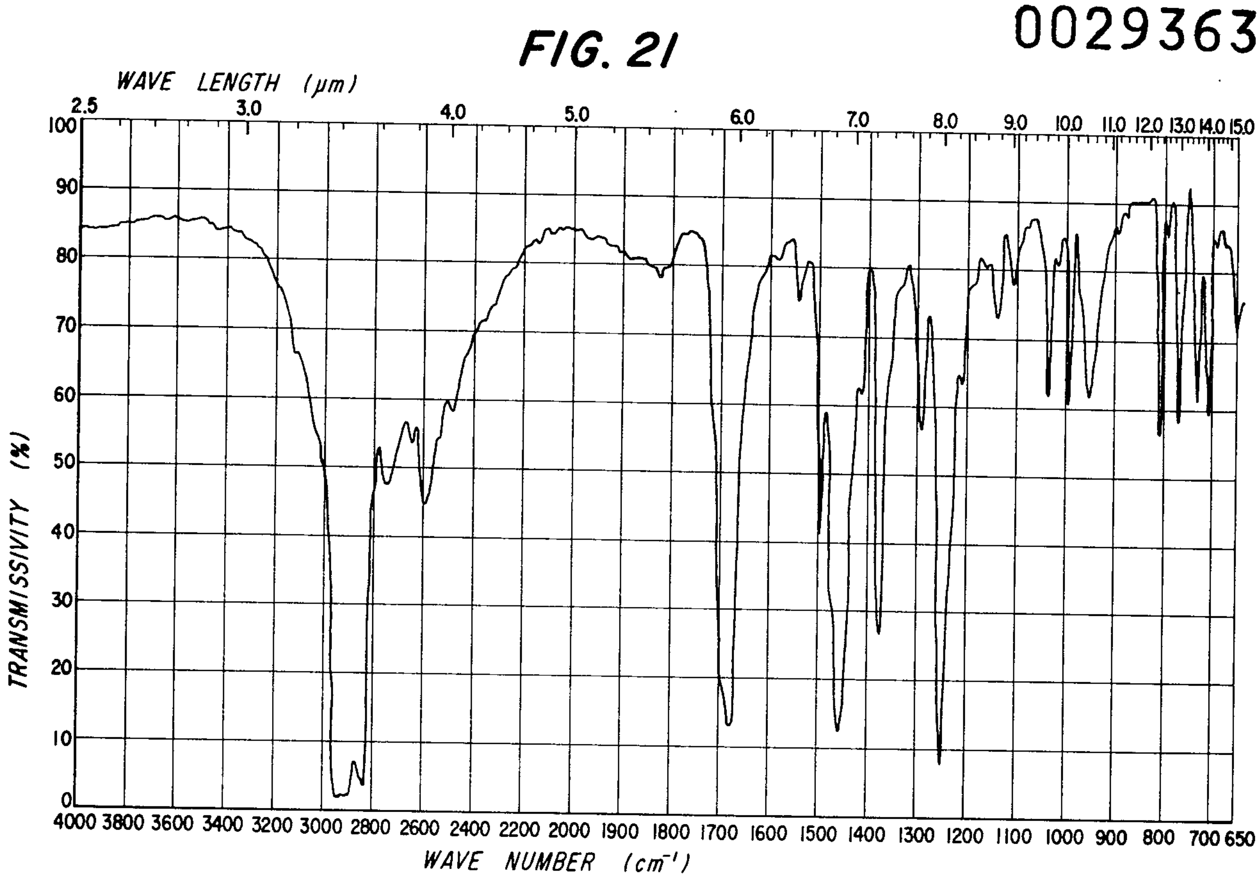
WAVE LENGTH (μm)
2.5 3.0 4.0 5.0 6.0 7.0 8.0 9.0 10.0 11.0 12.0 13.0 14.0 15.0
TRANSMISSIVITY (%)
100 90 80 70 60 50 40 30 20 10 0
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

FIG. 22

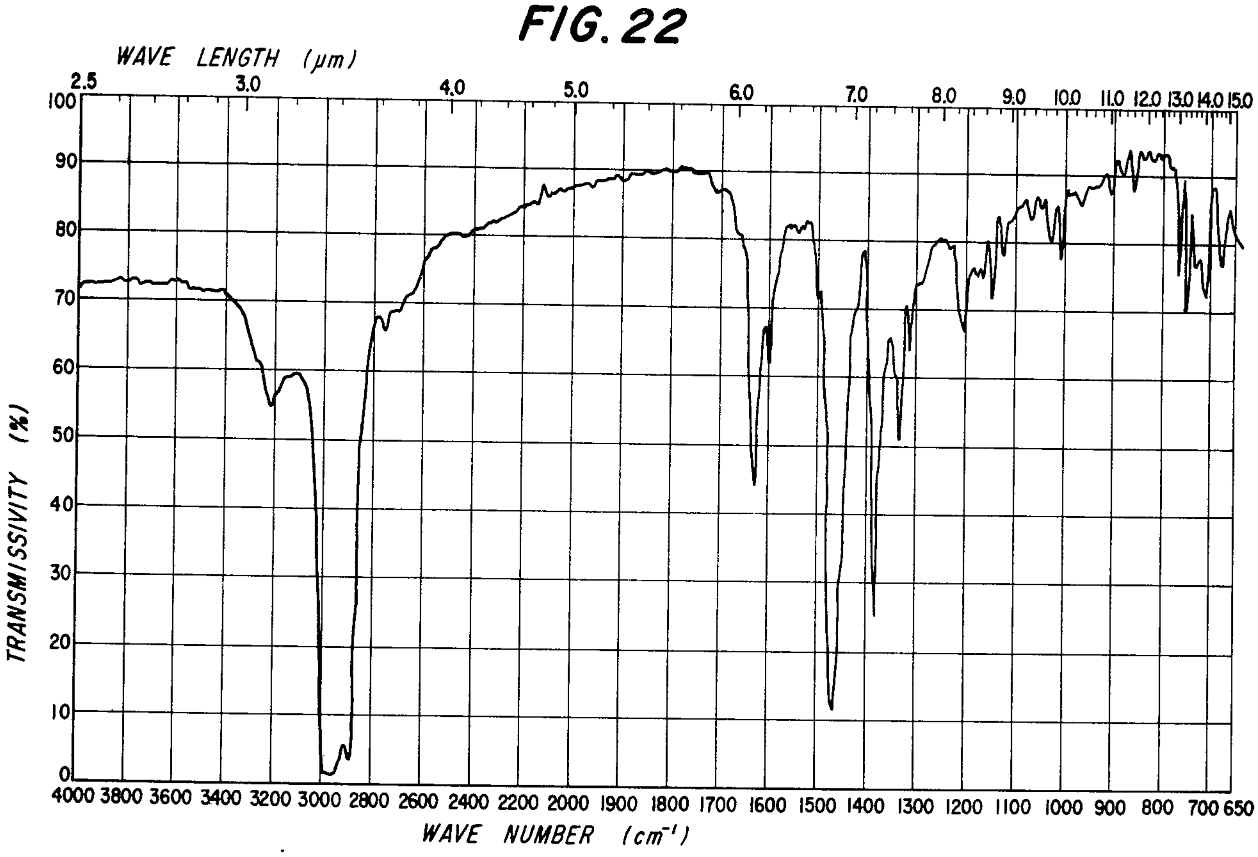
WAVE LENGTH (μm)
2.5 3.0 4.0 5.0 6.0 7.0 8.0 9.0 10.0 11.0 12.0 13.0 14.0 15.0
TRANSMISSIVITY (%)
100 90 80 70 60 50 40 30 20 10 0
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

0029363

FIG. 23

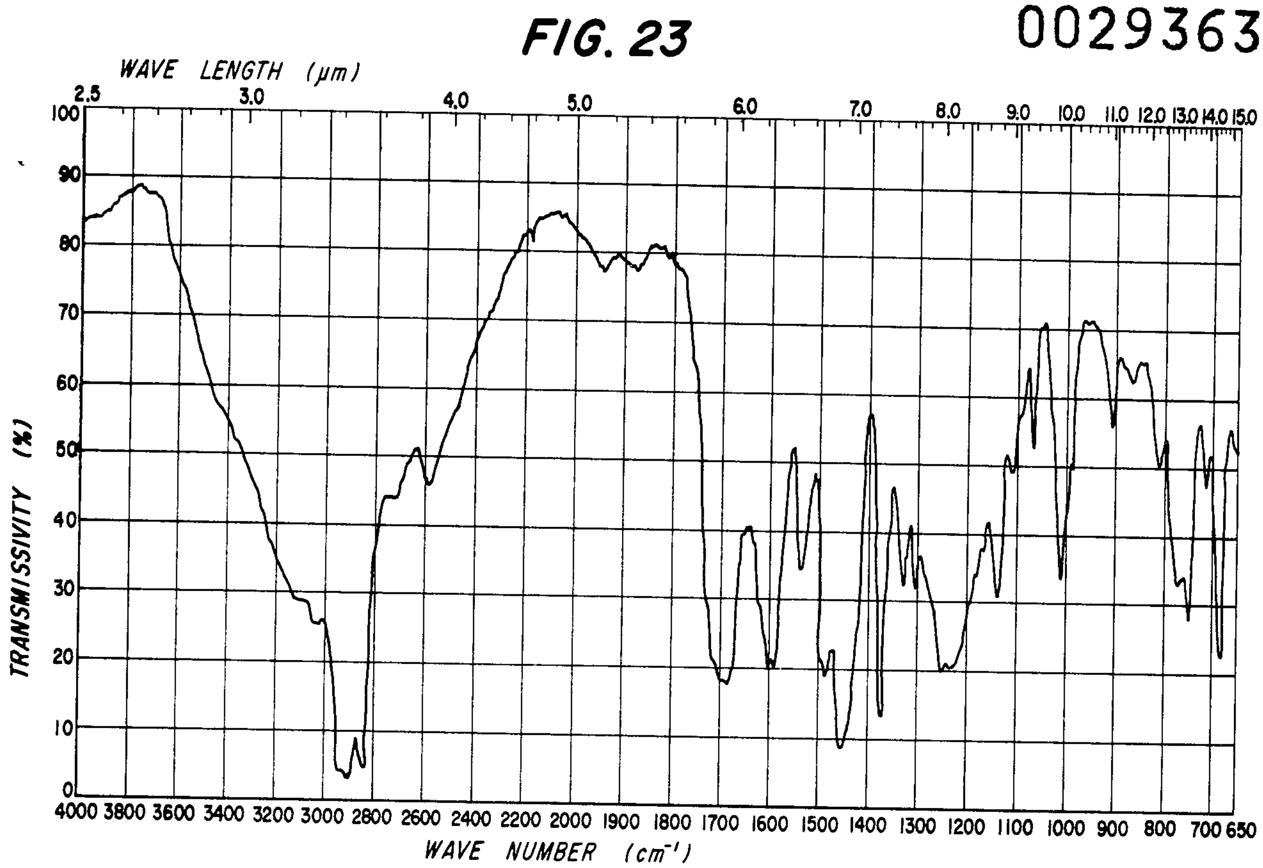


FIG. 24

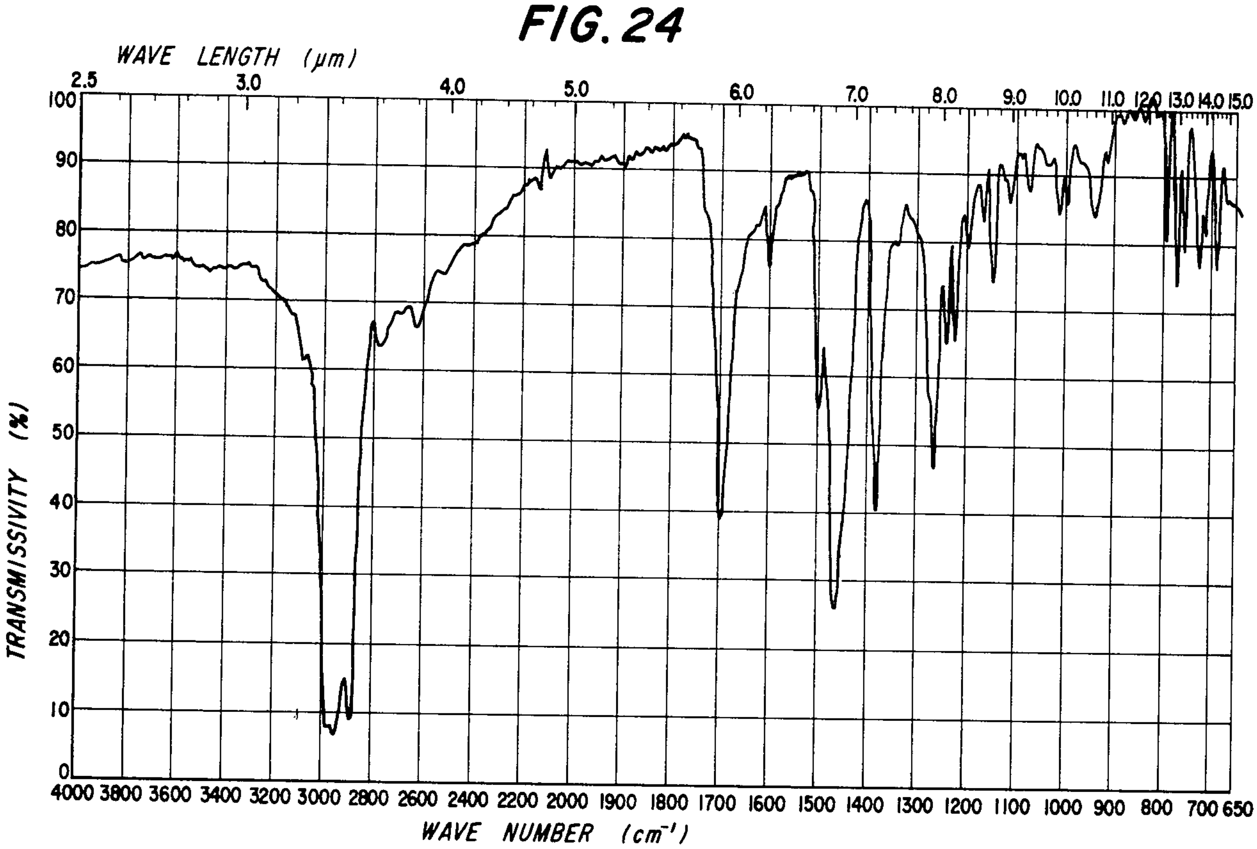

FIG. 25

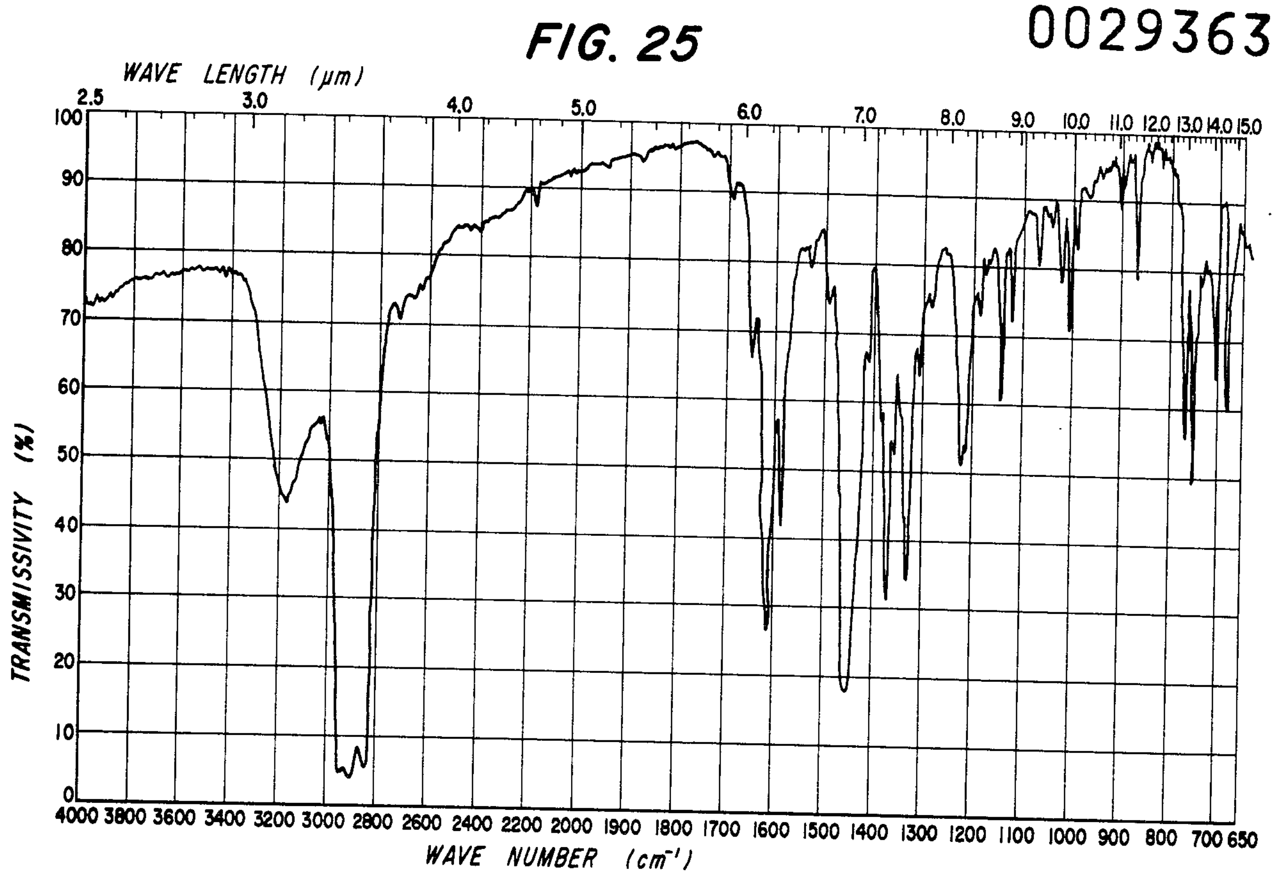
WAVE LENGTH (μm)
2.5
3.0
4.0
5.0
6.0
7.0
8.0
9.0
10.0
11.0
12.0
13.0
14.0
15.0
100
90
80
70
60
50
40
30
20
10
0
TRANSMISSIVITY (%)
4000
3800
3600
3400
3200
3000
2800
2600
2400
2200
2000
1900
1800
1700
1600
1500
1400
1300
1200
1100
1000
900
800
700
650
WAVE NUMBER (cm⁻¹)

European Patent Office

# EUROPEAN SEARCH REPORT

0029363

Application number

EP 80304106.0

## DOCUMENTS CONSIDERED TO BE RELEVANT

| Category | Citation of document with indication, where appropriate, of relevant passages | Relevant to claim |
|---|---|---|
| | GB - A - 1 048 104 (UPJOHN)<br>+ Totality +<br>-- | 1,9 |
| | US - A - 3 899 508 (WIKEL)<br>+ Columns 1,2 +<br>-- | 1,9 |
| | US - A - 3 449 350 (WALKER)<br>+ Abstract +<br>---- | 1,9 |

CLASSIFICATION OF THE APPLICATION (Int. Cl. 3)

C 07 D 231/14
C 07 D 231/12
A 61 K 31/415

TECHNICAL FIELDS SEARCHED (Int.Cl. 3)

C 07 D 231/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 10-02-1981 | LUX |

EPO Form 1503.1 06.78